# EUROPEAN PATENT SPECIFICATION

(11) **EP 0 718 277 B1**
(45) Date of publication and mention of the grant of the patent: **21.07.1999**
(21) Application number: 95120066.6
(22) Date of filing: 19.12.1995
(51) Int. Cl.: C07C 233/25, C07C 231/12, C07C 233/75, C07C 259/06, C07C 235/24, G03C 7/34

(54) **Method for producing 5-substituted-2-acylaminophenols, and 5-alkyl-2-acylaminophenol compound and N-acyl-N-(4-alkyl-phenyl)hydroxylamine compound**
Verfahren zur Herstellung von 5-substituierten 2-Acylaminophenolen, und 5-Alkyl-2-acylaminophenolverbindung und N-Acyl-N-(4-alkylphenyl)hydroxylaminverbindung
Procédé pour la préparation de 2-acylaminophénols substitués en position 5, et le composé 5-alkyl-2-acylaminophénol, et le composé N-acyl-N-(4-alkylphényl) hydroxylamine

(30) Priority: 20.12.1994 JP 33465094
(43) Date of publication of application: 26.06.1996
(73) Proprietor: FUJI PHOTO FILM CO., LTD., Kanagawa-ken, 250-01 (JP)
(72) Inventor: Sato, Tadahisa, c/o Fuji Photo Film Co., Ltd., Minami-ashigara-shi, Kanagawa-ken (JP)
(74) Representative: Hansen, Bernd, Dr. Dipl.-Chem.

(56) References cited:
- EP-A- 0 267 482
- DE-A- 3 527 116
- US-A- 5 006 660
- US-A- 5 026 631
- US-A- 5 214 194
- BULLETIN OF THE CHEMICAL SOCIETY OF JAPAN, vol. 55, no. 8, August 1982 pages 2681-2, Y. UCHIDA ET. AL. 'The Thermal Decomposition of N,O-Diacyl-N-t-butylhydroxylamines. IV. A Novel Acyloxy Migration in N,O-Diacyl-N-t-butylhydroxylamines.'

## Description

The present invention relates to a method for producing 2-acylaminophenols, as well as to 5-alkyl-2-acylaminophenol compounds produced by the same method and synthetic intermediates, N-acyl-N-(4-alkylphenyl) hydroxylamine compounds, and more particularly to 2-acylaminophenols that have a substituent in the 5-position, which 2-acylaminophenols are useful, for example, as synthetic intermediates for cyan couplers for silver halide color photography, for dye-releasing agents for instant color photography, and for medicines; a method for producing the same, and synthetic intermediates for that method.

### BACKGROUND OF THE INVENTION

2-acylaminophenols having a substituent in the 5-position are useful compounds as cyan couplers for silver halide color photography and as dye-releasing agents for instant color photography. Typical compounds thereof are 1 (cyan couplers described in JP-A ("JP-A" means unexamined published Japanese patent application) Nos. 9653/1986 and 39045/1986) and 2 (dye-releasing agents described in JP-A No. 16131/1981), and many studies for processes for the preparation of these compounds have been made.

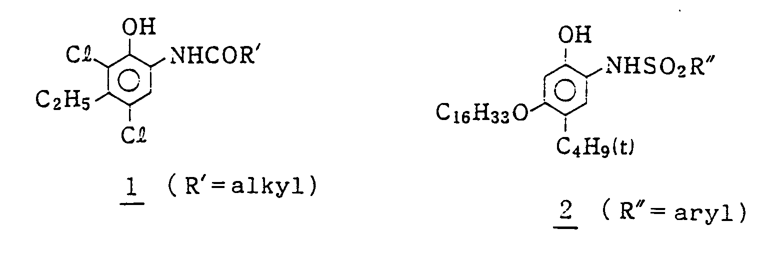

Major methods reported so far as synthetic methods for 1 are the followings:
(1) a method using, as a starting material, 4-chloro-5-ethylphenol.
   (1-a) 〈JP-A No. 57536/1986〉 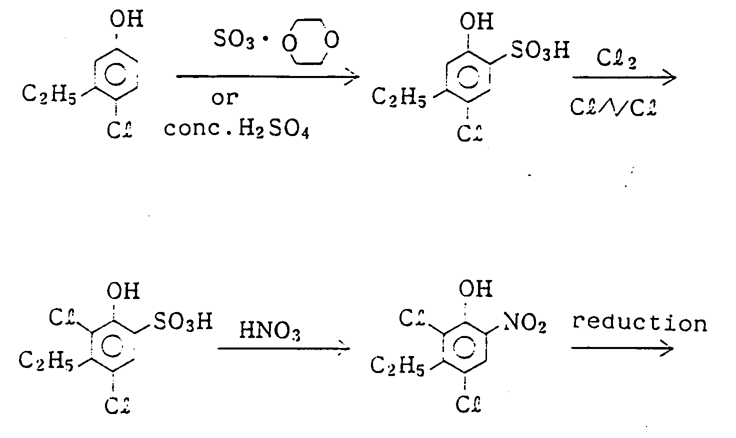 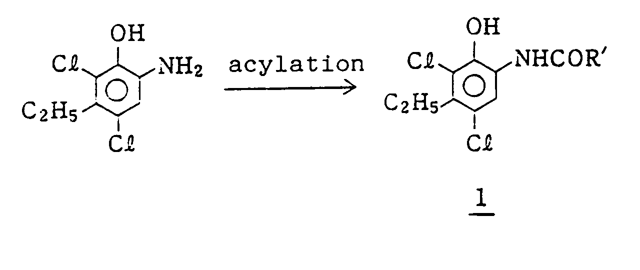
   (1-b) 〈JP-A Nos. 303958/1988, 32596/1993, and 32598/1993〉 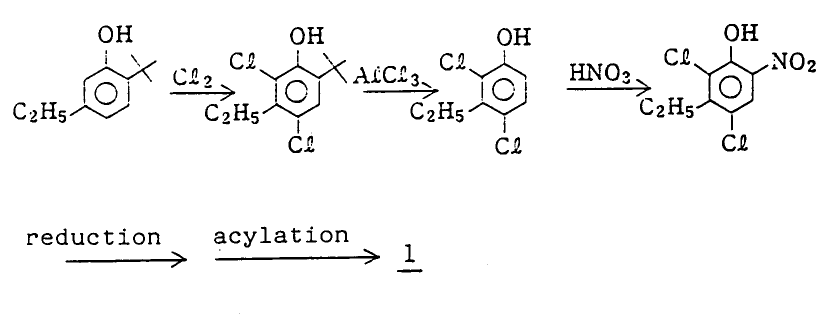
(2) a method using, as a starting material, 4-ethylnitrobenzene.
   (2-a) 〈JP-B("JP-B" means examined Japanese patent publication) No. 29780/1991, US 4,670,608, EP 175,151〉 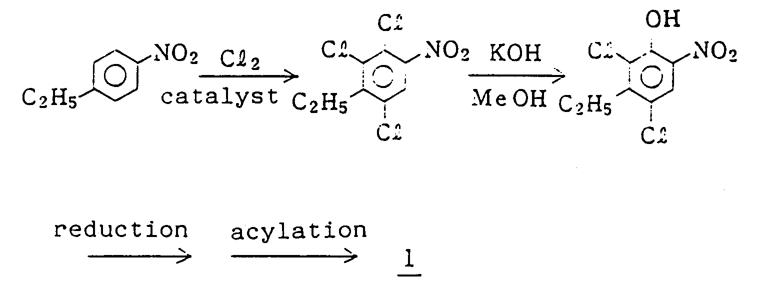
   (2-b) 〈JP-A No. 44552/1988〉 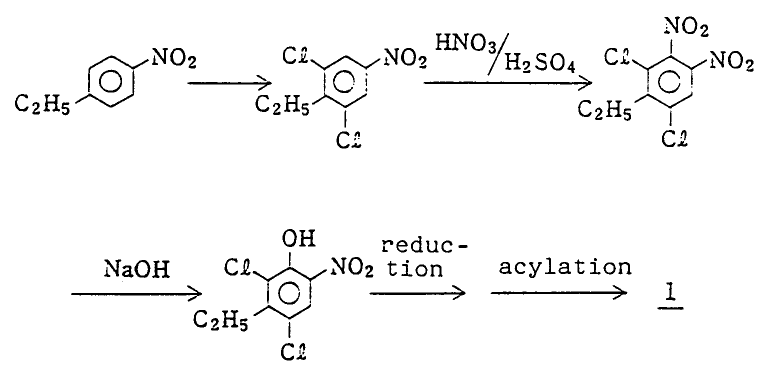
   (2-c) 〈US 5,214,194〉 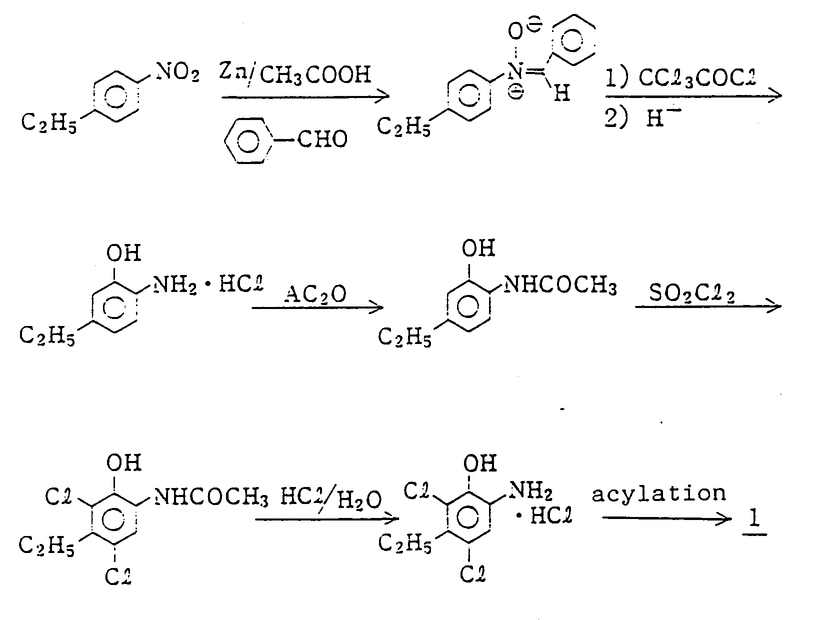
(3) a method using, as a starting material, 4-ethylbenzenesulfonic acid.
   (3-a) 〈JP-A No. 228943/1989〉 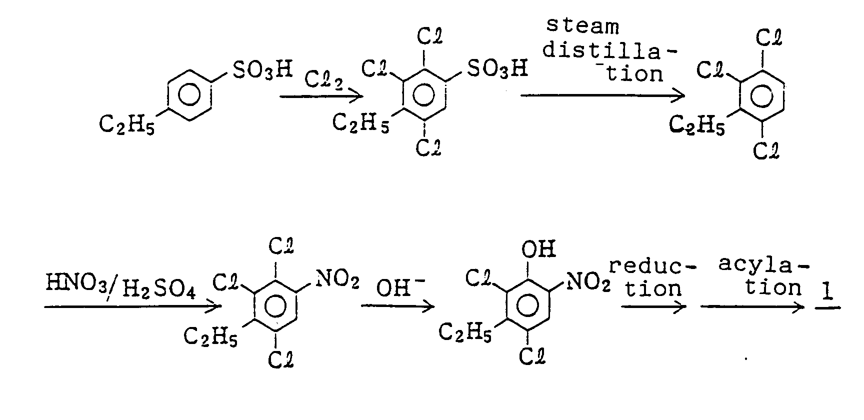
   (3-b) 〈JP-A No. 258649/1989〉

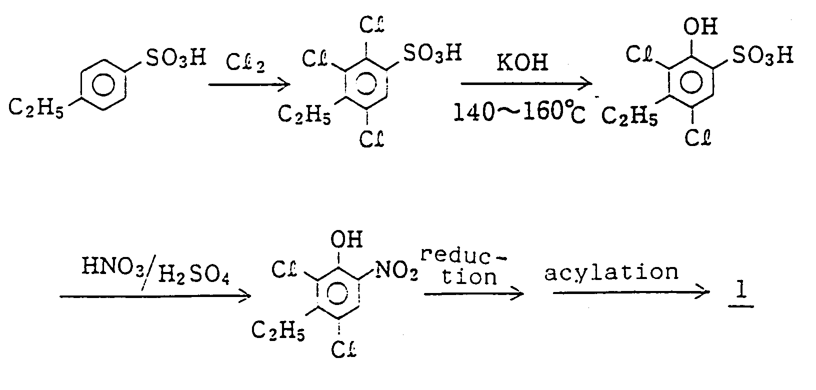

As a synthetic method for 2, the following method (JP-A 16131/1981) has been reported:

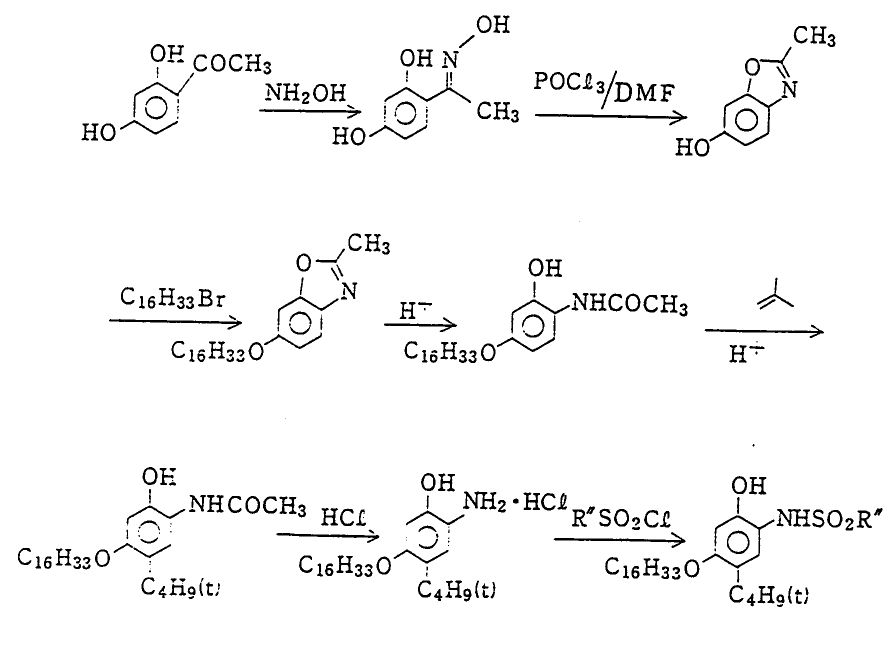

Although 1 and 2 can be produced industrially for the present by these synthetic methods, they involve many problems that should be improved upon.

With reference to the synthetic methods for 1, the method (1) has the problems that expensive 4-chloro-5-ethylphenol or its analogue is used, steps for protection and deprotection are required, and SO₃ and dichloroethane, which are harmful to the environment, are used. In the method (2), although the method (2) has a merit that inexpensive 4-ethylnitrobenzene is used as a starting material, the method has the problems that it is difficult to produce 4-ethyl-2,3,5-trichloronitrobenzene and 4-ethyl-3,5-dichloronitrobenzene in high purity and high yield, there is a safety concern regarding 2,6-dichloro-3,4-dinitroethylbenzene, and the production steps are numerous and complicated (in the case of (2-c)). In the method (3), although the starting raw materials are inexpensive, there are the problems that it is difficult to produce 4-ethyl-2,3,5-trichlorobenzenesulfonic acid highly selectively in high yield, in comparison with the method (2-a), which uses 4-ethylnitrobenzene, the number of the steps is large, and therefore the merits are few.

With reference to the synthetic method for 2, the starting material, 2',4'-acetophenone, is relatively expensive, and therefore it is desired to develop a method that can synthesize 2 from a more inexpensive starting material.

### SUMMARY OF THE INVENTION

An object of the present invention is to solve the problems involved in the synthesis of 1 and 2, i.e., to develop a method for synthesizing 2-acylaminophenols that (1) uses inexpensive starting materials, (2) has short steps, (3) is environmentally friendly, (4) is high in safety, and (5) is low in cost. If these problems are solved, it is considered that the probability of the use of 5-substituted-2-acylaminophenols as synthetic intermediates, for example, for silver halide color photography, instant color photography, and medical supplies, can be increased greatly.

Other and further objects, features, and advantages of the invention will appear more evident from the following description.

### DETAILED DESCRIPTION OF THE INVENTION

The present inventor has studied intensely in various ways to solve the above problems. As a result, I have found that 2-acylaminophenols having a substituent, such as an aliphatic hydrocarbon group, in the 5-position, can be obtained with good selectivity by the following method. That is, the method comprises preparing a benzene compound having an acyloxy group and an acylamino group in the 3- and 4-positions, respectively, toward the substituent (such as an aliphatic hydrocarbon group) introduced onto the ring, by the acyloxy rearrangement reaction of N,O-diacyl-N-(4-substituted phenyl)hydroxylamine, and then subjecting the benzene compound to a deacylation reaction, to cause only the acyloxy group to be deacylated. This finding has led to the present invention.

The present invention herein provided is:
(1) a method for producing 5-substituted-2-acylaminophenols represented by the following formula (I), comprising partially deacylating a compound represented by the following formula (V): 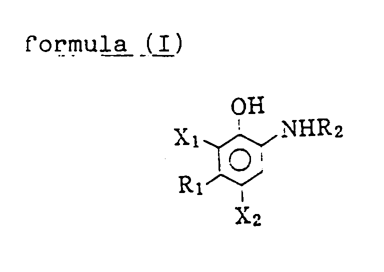 in formula (I), R₁ represents an aliphatic hydrocarbon group, an aliphatic hydrocarbon oxy group, an aryloxy group, an acylamino group, a disubstituted amino group, or a hydroxyl group; R₂ represents an acyl group; and X₁ and X₂ each represent a hydrogen atom or a halogen atom, 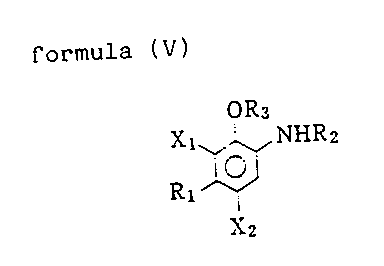 wherein R₁, R₂, X₁, and X₂ each have the same meaning as defined in formula (I), and R₃ represents an acyl group;
(2) the method as stated in the above (1), wherein the deacylation is carried out in the presence of hydroxylamine;
(3) the method as stated in the above (1) or (2), wherein the compound represented by formula (V) is a compound obtained by subjecting a compound represented by the following formula (IV) to acyloxyl rearrangement: 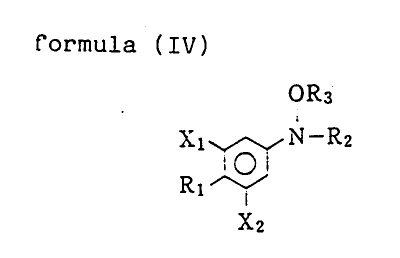 in formula (IV), R₁, R₂, R₃, X₁, and X₂ each have the same meaning as defined in formula (V);
(4) the method as stated in the above (1), (2), or (3), wherein the said acyloxyl rearrangement is carried out in the presence of acid catalyst(s);
(5) the method as stated in the above (3), or (4), wherein the compound represented by formula (IV) is a compound obtained by acylating N-acyl-N-substituted phenylhydroxylamines represented by formula (II): 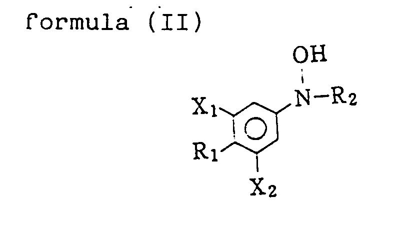 in formula (II), R₁, R₂, X₁, and X₂ each have the same meaning as defined in formula (IV);
(6) a method for producing a compound represented by the following formula (XI), comprising acylating a compound represented by the following formula (IX), to form a compound represented by the following formula (X), and subjecting the compound of formula (X) to an acyloxyl rearrangement reaction to give a compound of formula (XI): 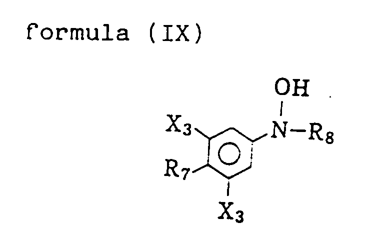 wherein R₇ represents an aliphatic hydrocarbon group or an aliphatic hydrocarbon oxy group having 2 to 18 carbon atoms, or an acylamino group; R₈ represents an acyl group; and X₃ represents a hydrogen atom or a halogen atom, 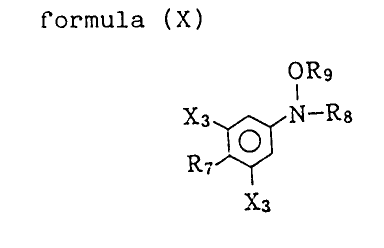 wherein R₇, R₈, and X₃ each have the same meaning as defined in formula (IX), and R₉ represents an acyl group, 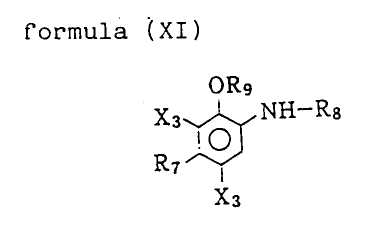 wherein R₇, R₈, R₉, and X₃ each have the same meaning as defined in formulae (IX) and (X);
(8) an N-acyl-N-(4-alkylphenyl)hydroxylamine compound represented by the following formula (VIII): 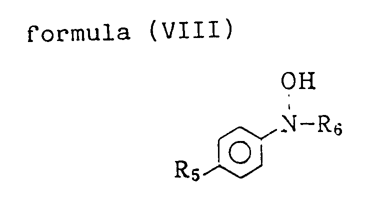 wherein R₅ represents an alkyl group having 2 to 9 carbon atoms, and R₆ represents an aliphatic carbonyl group having 15 to 22 carbon atoms.

First, the substituents R₁, R₂, X₁, and X₂ of the compounds represented by formulae (I) to (V) are described in detail.

R₁ represents an aliphatic hydrocarbon group, an aliphatic hydrocarbon oxy group, an aryloxy group, an acylamino group, a disubstituted amino group, or a hydroxyl group; and those groups, except the hydroxyl group, are described in detail. The aliphatic hydrocarbon group represents a straight-chain or branched-chain alkyl, aralkyl, alkenyl, alkynyl, cycloalkyl, or cycloalkenyl group having 1 to 32 carbon atoms; the aliphatic hydrocarbon oxy group represents a straight-chain or branched-chain alkoxy, cycloalkyloxy, aralkyloxy, alkenyloxy, cycloalkenyloxy, or alkynyloxy group having 1 to 32 carbon atoms; the aryloxy group represents a phenoxy or naphthoxy group having 6 to 23 carbon atoms; the acylamino group represents an alkanoylamino, aryloylamino, alkanesulfonylamino, or allenesulfonylamino group having 1 to 32 carbon atoms; and the disubstituted amino group represents an N,N-dialkylamino, N-alkyl-N-arylamino, N,N-diarylamino, N-alkyl-N-acylamino, or N-aryl-N-acylamino group having 2 to 32 carbon atoms. These groups may be substituted, and the substituent includes a halogen atom and an organic substituent bonded through a carbon atom, a silicon atom, an oxygen atom, a nitrogen atom, or a sulfur atom. Particular examples of the substituent include an alkyl group, an alkenyl group, an alkynyl group, an aryl group, a heterocyclic group, a cyano group, an acyl group, an aryloxycarbonyl group, an alkoxycarbonyl group, a carbamoyl group, an alkoxy group, an aryloxy group, a heterocyclic oxy group, an acyloxy group, a carbamoyloxy group, a silyloxy group, a silyl group, an aryloxycarbonylamino group, an acylamino group, an anilino group, a ureido group, a sulfamoylamino group, an alkoxycarbonylamino group, a sulfonamido group, an imido group, an alkylthio group, an arylthio group, a heterocyclic thio group, a sulfamoyl group, a sulfonyl group, a sulfinyl group, a fluorine atom, a chlorine atom, a bromine atom, and an iodine atom.

More particularly, besides a hydroxyl group, R₁ includes, for example, an aliphatic hydrocarbon group, such as methyl, ethyl, propyl, isopropyl, n-butyl, t-butyl, cyclopropyl, cyclopentyl, cyclohexyl, 3-cyclohexenyl, 2-propenyl, 2-propynyl, benzyl, methoxyethyl, ethoxyethyl, t-butoxyethyl, phenoxyethyl, methanesulfonylethyl, 2-hydroxyethyl, 3-(3-pentadecylphenoxy)propyl, 4,4,4-trifluorobutyl, and 3-(2,4-di-aminophenoxy)propyl; an aliphatic hydrocarbon oxy group, such as methoxy, ethoxy, isopropoxy, n-butyloxy, t-butyloxy, octyloxy, dodecyloxy, hexadecyloxy, 2-methoxyethoxy, 2-ethoxyethoxy, 2-dodecyloxyethoxy, 2-phenoxyethoxy, 3-cyclohexenyloxy, 2-propenyloxy, 2-propionyloxy, benzyloxy, 2-(4-t-amylphenoxy)ethoxy, 2-(4-nitrophenoxy)ethoxy, and 2-(2,4-dichlorophenoxy)ethoxy; an aryloxy group, such as phenoxy, 2-methoxyphenoxy, 2-methylphenoxy, 3-methoxyphenoxy, 3-methylphenoxy, 4-methoxyphenoxy, 4-methylphenoxy, 2,4-dimethoxyphenoxy, 2,4-dimethylphenoxy, 4-methoxycarbonylphenoxy, 1-naphthyloxy, 2-naphthyloxy, 1-methoxy-2-naphthyloxy, 2-methoxy-1-naphthyloxy, and 4-methoxy-1-naphthyloxy; an acylamino group, such as acetylamino, propionylamino, butyrylamino, isobutyrylamino, pivaloylamino, octanoylamino, palmitoylamino, stearoylamino, oleylamino, 3-(4-nitrophenyl)butanoyl, 2-(2,4-di-t-amylphenoxy)hexanoylamino, 2-(2,4-di-t-amylphenoxy)butyrylamino, 2-(2-chlorophenoxy)tetradecanoylamino, 2-(2-chloro-4-t-amylphenoxy)octanoylamino, 2-(2-cyanophenoxy)octanoylamino, 2-(2,4-di-t-amylphenoxy)-2-methylbutyrylamino, 2-(hexadecanesulfonyl)-2-methylbutyrylamino, methanesulfonylamino, ethanesulfonylamino, hexadecanesulfonylamino, benzenesulfonylamino, 2-octyloxy-5-t-octylbenzenesulfonylamino, and 4-dodecyloxybenzenesulfonylamino; and a disubstituted amino group, such as dimethylamino, diethylamino, dioctylamino, octylmethylamino, dodecylmethylamino, N-methylphenylamino, N-methylacetylamino, N-methyloctanoylamino, and N-methylbenzoylamino.

Preferably, R₁ represents an aliphatic hydrocarbon group, an aliphatic hydrocarbon oxy group, or an acylamino group; more preferably an alkoxy or alkyl group having 2 to 20 carbon atoms; particularly preferably an alkyl group having 2 to 9 carbon atoms; and most preferably an ethyl group inter alia.

R₂ represents an acyl group, and more particularly a formyl group, an aliphatic carbonyl group having 2 to 36 carbon atoms, an aromatic carbonyl group having 7 to 36 carbon atoms, an alkyloxycarbonyl group having 2 to 36 carbon atoms, an aryloxycarbonyl group having 7 to 36 carbon atoms, an N-alkylcarbamoyl group having 2 to 36 carbon atoms, an N-arylcarbamoyl group having 7 to 36 carbon atoms, an alkanesulfonyl group having 1 to 36 carbon atoms, an arenesulfonyl group having 6 to 36 carbon atoms, or a phosphinoyl group having 2 to 36 carbon atoms. In this circumstance, the aliphatic carbonyl group represents an alkanoyl group, that is, it represents a carbonyl group of a saturated aliphatic hydrocarbon; and an alkenoyl or alkynolyl group, that is, it represents a carbonyl group of an unsaturated aliphatic hydrocarbon. Also in this circumstance, the aromatic carbonyl group represents an aryloyl group; that is, it represents a carbonyl group of an aromatic hydrocarbon; and an aromatic heterocyclic carbonyl group. These acyl groups may have substituents that the above R₁ may have.

More particularly, R₂ represents a formyl group, an aliphatic carbonyl group, such as acetyl, propionyl, pivaloyl, octanoyl, lauroyl, palmitoyl, stearyl, isostearoyl, eicosanoyl, docosanoyl, hexaconoyl, oleyl, perfluorobutyryl, 2-(2,4-di-t-amylphenoxy)butyryl, 2-(2,4-di-t-amylphenoxy)acetyl, 2-(2,4-di-t-amylphenoxy)hexanoyl, 4-(2,4-di-t-amylphenoxy)butyryl, 2-(2,4-di-t-butylphenoxy)butyryl, acryloyl, methacryloyl, or propiolyl; an aromatic carbonyl group, such as benzoyl, 2-chlorobenzoyl, 4-chlorobenzoyl, 4-nitrobenzoyl, 2-nitrobenzoyl, 3-nitrobenzoyl, 4-methoxybenzoyl, 2-methoxybenzoyl, 4-t-butylbenzoyl, 4-acetylaminobenzoyl, 4-chloro-3-nitrobenzoyl, teterafluorobenzoyl, 2-furoyl, 3-pyridinecarbonyl, 4-pyridazinecarbonyl, or 2-thiophenylcarbonyl; an alkyloxycarbonyl group, such as methoxycarbonyl, ethoxycarbonyl, and octyloxycarbonyl; an aryloxycarbonyl group, such as phenoxycarbonyl, 4-nitrophenoxycarbonyl, 4-methoxyphenoxycarbonyl, and 4-chlorophenoxycarbonyl; an N-alkylcarbamoyl group, such as N-methylcarbamoyl, N,N-dimethylcarbamoyl, and N-octylcarbamoyl; an N-arylcarbamoyl group, such as N-phenylcarbamoyl, N-(4-cyanophenyl)carbamoyl, N-(4-chloro-3-cyanophenyl)carbamoyl, N-(4-fluorophenyl)carbamoyl, N-(3,4-dicyanophenyl)carbamoyl, and N-(4-butanesulfonylphenyl)carbamoyl; an alkanesulfonyl group, such as methanesulfonyl, ethanesulfonyl, butanesulfonyl, and dodecanesulfonyl; an arenesulfonyl, such as benzenesulfonyl, p-toluenesulfonyl, 1-naphthalenesulfonyl, 2-nitrobenzenesulfonyl, 3-nitrobenzenesulfonyl, 4-nitrobenzenesulfonyl, 2-chloro-5-nitrobenzenesulfonyl, 4-chloro-3-nitrobenzenesulfonyl, 4-octyloxybenzenesulfonyl, 4-palmitoylaminobenzenesulfonyl, and 2-butyloxy-4-t-octylbenzenesulfonyl; and a phosphinoyl group, such as dimethylphosphinoyl, dibutylphosphinoyl, diethoxyphosphinoyl, bis(dimethylamino)phosphinoyl, diphenylphosphinoyl, and diphenoxyphosphinoyl.

R₂ preferably represents an aliphatic carbonyl group or an aromatic carbonyl group, and more preferably an unsubstituted alkanoyl group or a substituted or unsubstituted aryloyl group.

Particularly preferably R₂ represents an unsubstituted alkanoyl group having 15 to 22 carbon atoms, and most preferably a palmitoyl group.

X₁ and X₂ each represent a hydrogen atom or a halogen atom, and the halogen atom is particularly a fluorine, chlorine, bromine, or iodine atom. X₁ and X₂ may be different from each other, but preferably X₁ and X₂ represent the same atom. Preferably X₁ and X₂ each represent a hydrogen atom or a chlorine atom, more preferably a hydrogen atom.

R₃ represents an acyl group that is the same as that of R₂, and preferably R₃ represents -C(=O)R₄ or -SO₂R₁₀, wherein R₄ represents a hydrogen atom, an aliphatic hydrocarbon group, an aryl group, an aliphatic hydrocarbon oxy group, an aryloxy group, an acyl group, or a substituted amino group, and R₁₀ represents an aliphatic hydrocarbon group, an aryl group, or a hydroxyl group.

More particularly, R₄ represents a hydrogen atom, an aliphatic hydrocarbon group, an aryl group, an aliphatic hydrocarbon oxy group, an aryloxy group, an acyl group, or a substituted amino group; and besides a hydrogen atom, R₄ includes specifically an aliphatic hydrocarbon group having 1 to 26 carbon atoms, such as methyl, ethyl, vinyl, monochloromethyl, dichloromethyl, trichloromethyl, trifluoromethyl, perfluoroethyl, or perfluoropropyl; an aryl group having 6 to 32 carbon atoms, such as phenyl, naphthyl, o-nitrophenyl, p-nitrophenyl, m-nitrophenyl, p-fluorophenyl, 3,4-dichlorophenyl, 3,4-dinitrophenyl, pentachlorophenyl, perfluorophenyl, p-cyanophenyl, or p-ethoxycarbonylphenyl; an aliphatic hydrocarbon oxy group having 1 to 30 carbon atoms, such as methoxy, ethoxy, propoxy, 2,2,2-trichloroethoxy, 2,2,2-trifluoroethoxy, or benzyloxy; an aryloxy group having 6 to 32 carbon atoms, such as phenoxy, naphthoxy, p-nitrophenoxy, p-ethoxycarbonylphenoxy, p-cyanophenoxy, or pentachlorophenoxy; an acyl group having 1 to 26 carbon atoms, such as acetyl, chlorocarbonyl, ethoxycarbonyl, benzoyl, or trifluoroacetyl; and a substituted amino group having 1 to 32 carbon atoms, such as methylamino, dimethylaminoanilino, N-methylanilino, pyrrolidino, morpholino, and imidazolino.

Preferably R₄ represents an alkyl group or an aryl group, more preferably an alkyl group, such as methyl, monochloromethyl, dichloromethyl, or trichloromethyl; or a phenyl group, such as phenyl, p-nitrophenyl, m-nitrophenyl, p-chlorophenyl, or p-fluorophenyl.

R₁₀ represents an aliphatic hydrocarbon group, an aryl group, or a hydroxyl group. Particularly the aliphatic hydrocarbon group is one having 1 to 26 carbon atoms, such as methyl, trifluoromethyl, or ethyl; and the aryl group is one having 6 to 32 carbon atoms, such as phenyl, tolyl, m-nitrophenyl, or 3-nitro-4-chlorophenyl.

Preferably R₁₀ represents a methyl group, a phenyl group, or a tolyl group, and particularly preferably a methyl group.

Particularly preferable structures of formulae (I) and (II) can be specifically shown by the following general formulae (VII) and (VIII). Compounds represented by formula (VIII) are compounds that do not appear in literature: 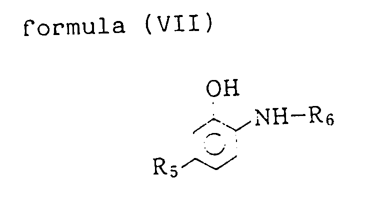 wherein R₅ represents an alkyl group having 2 to 9 carbon atoms, and R₆ represents an aliphatic carbonyl group having 15 to 22 carbon atoms, 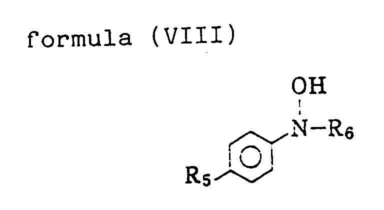 wherein R₅ and R₆ have the same meanings as defined in formula (VII).

R₅ and R₆ are now described in more detail. R₅ represents an alkyl group having 2 to 9 carbon atoms, and specific examples are an ethyl group, a propyl group, an isopropyl group, an n-butyl group, a t-butyl group, an n-pentyl group, an n-hexyl group, an n-heptyl group, an n-octyl group, or an n-nonyl group. R₆ represents an aliphatic carbonyl group having 15 to 22 carbon atoms, and specific examples are a palmitoyl group, an isopalmitoyl group, a stearoyl group, an isostearoyl group, an eicosanoyl group, an isoeicosanoyl group, a docosanoyl group, a hexacosanoyl group, an isohexacosanoyl group, or an oleyl group.

The long chain of R₆ having 15 to 22 carbon atoms of the compound represented by formula (VII) makes the compound have a highly lipophilic nature and high diffusion resistance. This leads to a great advantage that the compound as it is can be used as a photographic cyan coupler or as a compound for a photographic useful agent.

Preferably, R₅ represents an ethyl group, and R₆ represents a palmitoyl group.

A preferable combination of R₅ with R₆ is such that, when R₅ represents an ethyl group, R₆ represents a palmitoyl group, a stearoyl group, a docosanoyl group, or a hexacosanoyl group; whereas when R₆ represents a palmitoyl group, R₅ represents an ethyl group, a propyl group, an isopropyl group, or a t-butyl group.

The most preferable combination of R₅ with R₆ is such that R₅ represents an ethyl group and R₆ represents a palmitoyl group.

The synthetic method of the present invention is useful not only in synthesizing 5-substituted-2-aminophenols but also in synthesizing O,N-diacyl-5-alkyl, or alkoxyaminophenols, which are intermediates of the present synthetic method and are considered useful for various purposes.

The synthetic method is shown below:

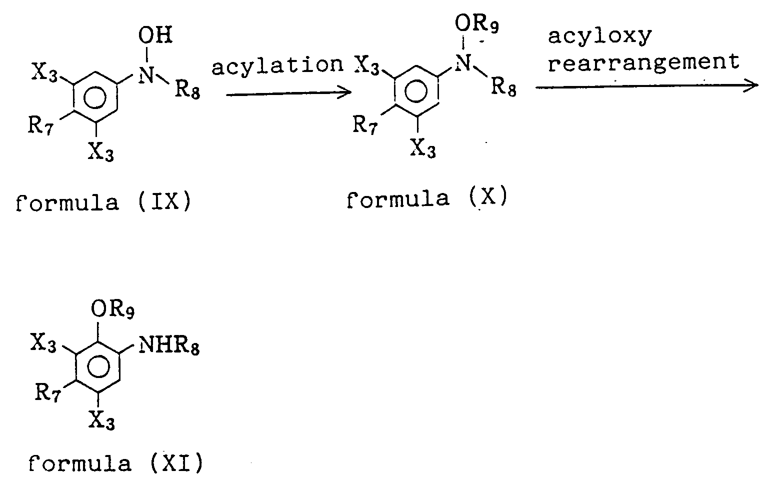

Substituents R₇, R₈, R₉, and X₃ of formulae (IX), (X), and (XI) given above are described now.

R₇ represents an aliphatic hydrocarbon group or an aliphatic hydrocarbon oxy group having 2 to 18 carbon atoms, or an acylamino group, and particularly the groups R₇ represents are the same as those described for R₁ of formula (I) and are selected from them.

The following are preferable: the aliphatic hydrocarbon group of R₇ is the same as that of R₅ of formula (VII); the aliphatic hydrocarbon oxy group of R₇ is an alkoxy group having 2 to 18 carbon atoms; and the acylamino group of R₇ is an alkanoylamino group having 1 to 18 carbon atoms.

Particularly preferably R₇ is an ethyl group, a dodecyloxy group, or an acetylamino group.

R₈ and R₉ each represent an acyl group, and particularly the acyl group is one of the groups described for R₂ of formula (I) and is selected from them.

Preferably R₈ and R₉ each represent an aliphatic carbonyl group having 1 to 22 carbon atoms, an aromatic carbonyl group, an alkanesulfonyl group, or an arenesulfonyl group.

Particularly preferable groups represented by R₈ and R₉ are such that R₈ represents an aliphatic carbonyl group having 15 to 22 carbon atoms, and R₉ represents an α-haloalkanoyl group, an aryloyl group, an alkanesulfonyl group having 1 to 7 carbon atoms, or an arenesulofonyl group.

X₃ represents a hydrogen atom or a halogen atom, preferably a hydrogen atom or a chlorine atom, and particularly preferably a hydrogen atom.

Parenthetically, in formulae (I) to (XI), the number of carbon atoms of the groups means the total number of carbon atoms of the particular group plus carbon atoms of the substituent on the particular group, if any.

Specific examples of the compounds represented by formulae (I), (II), and (V) (represented by the following formulae (V-A) and (V-B)) of the present invention are shown below, but the present invention is not restricted to them.

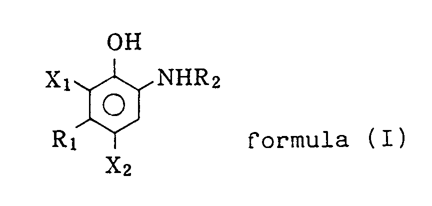

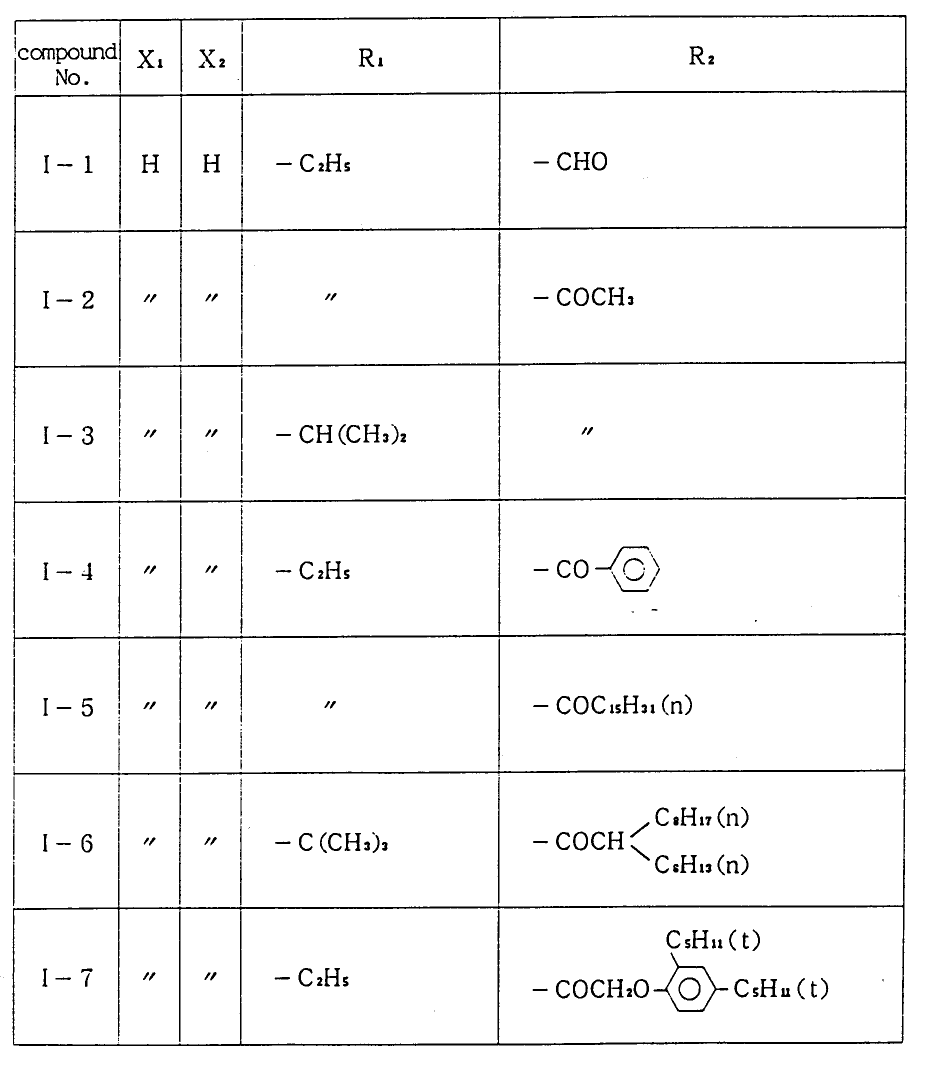

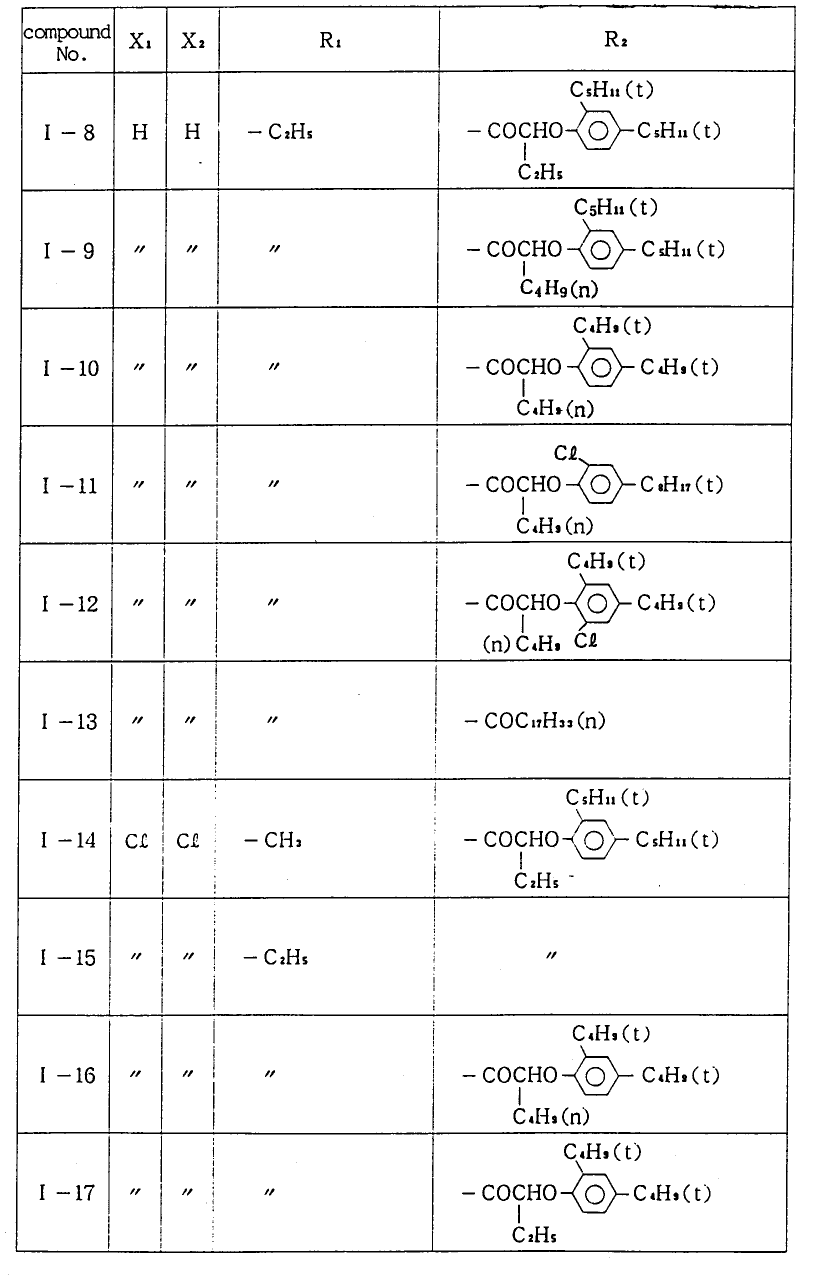

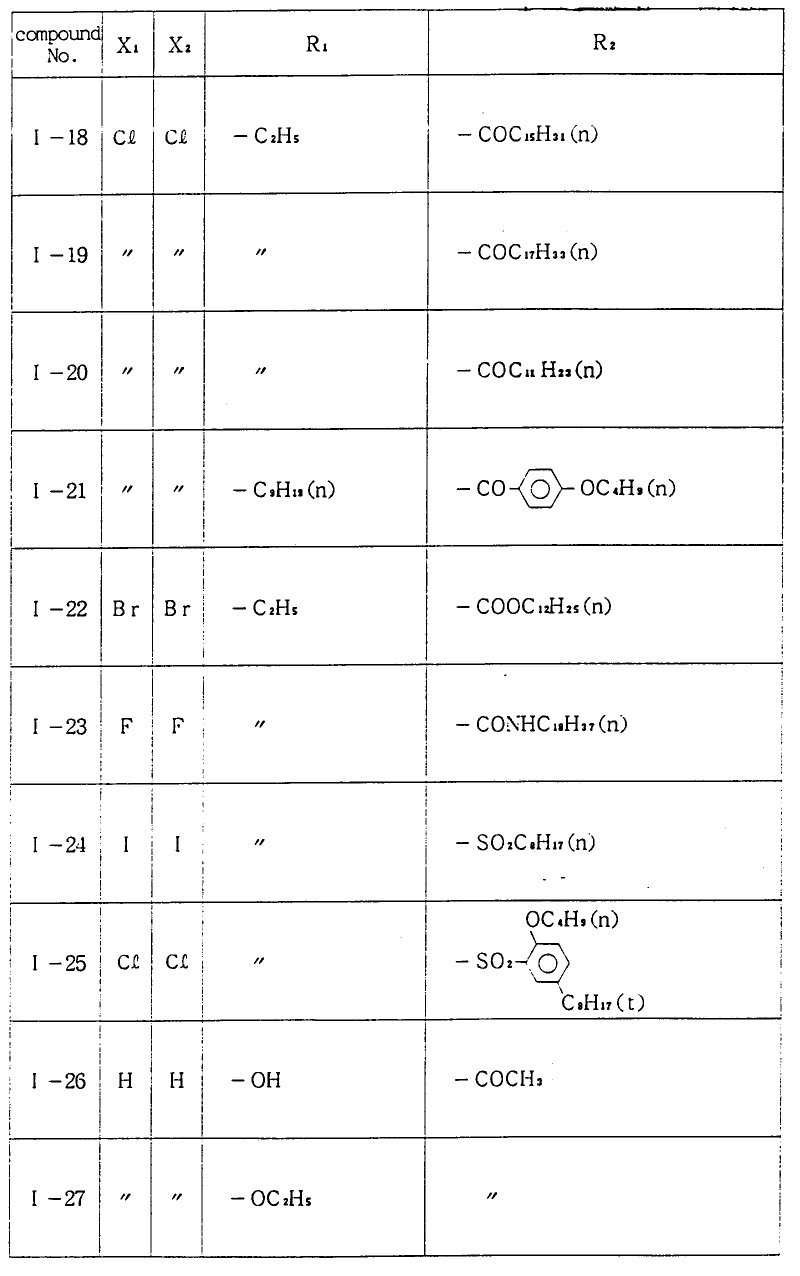

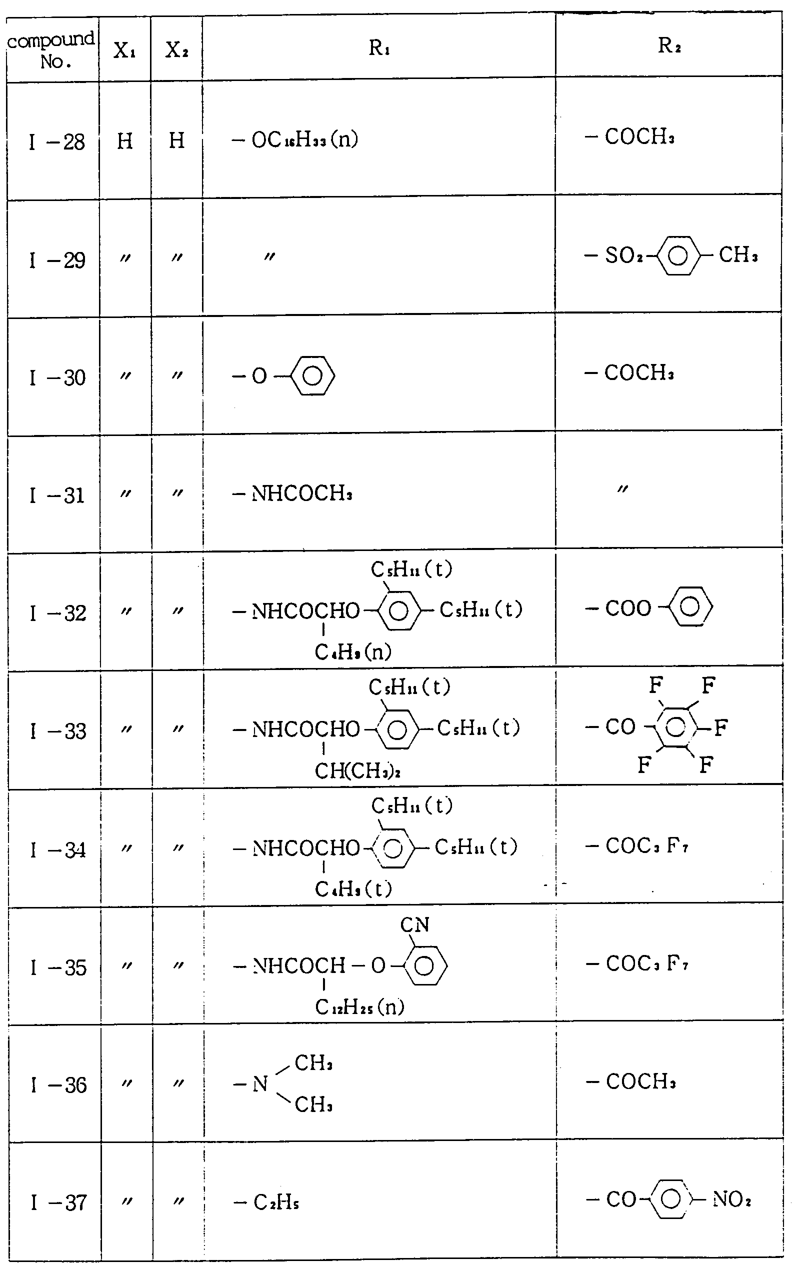

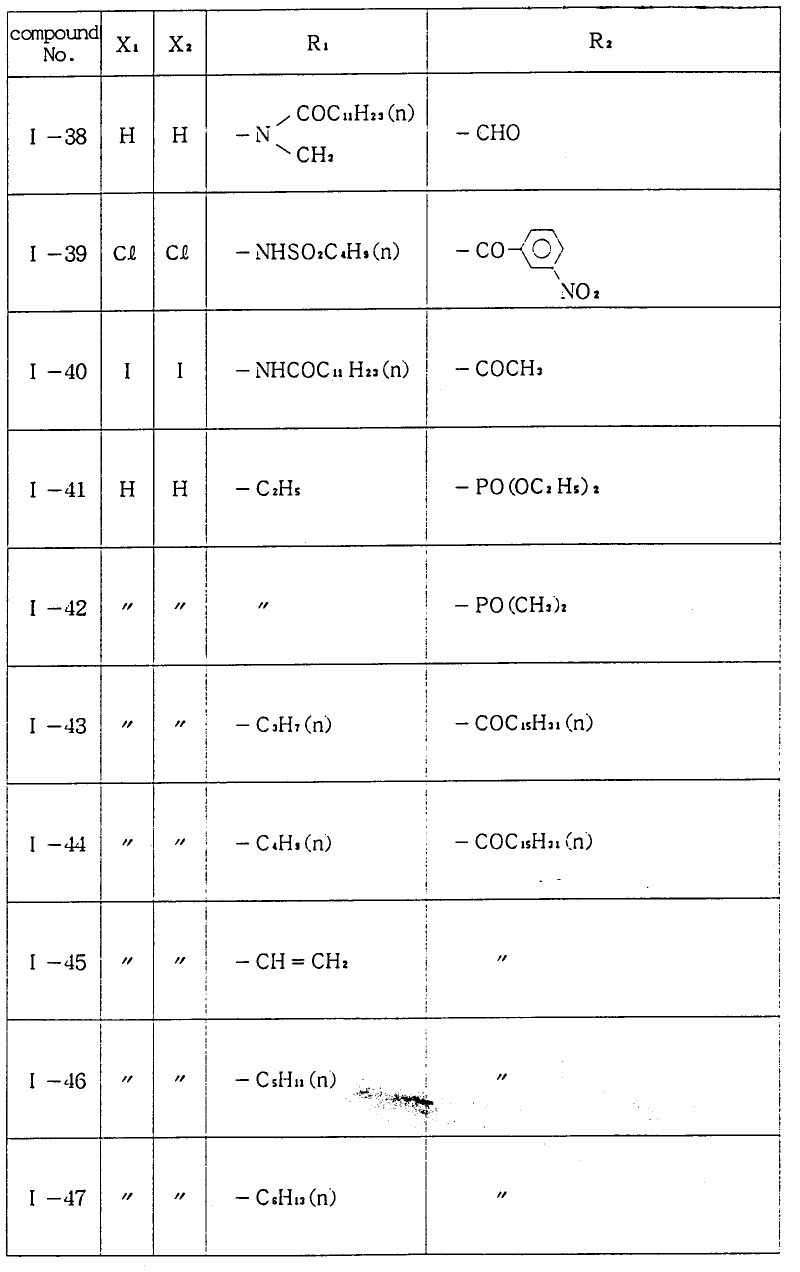

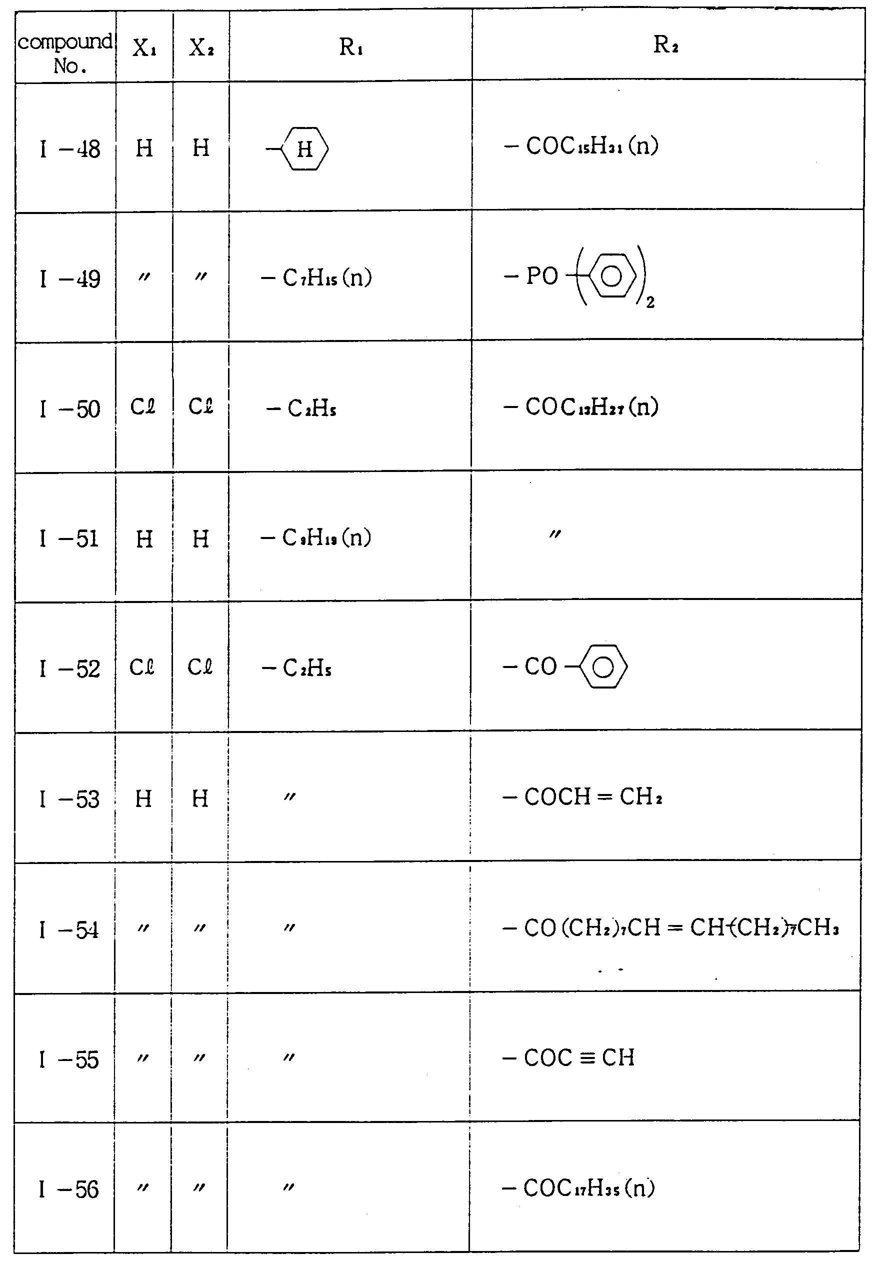

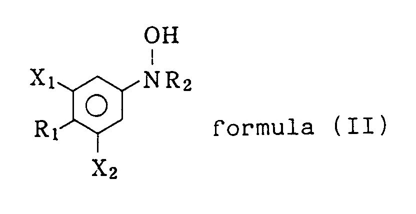

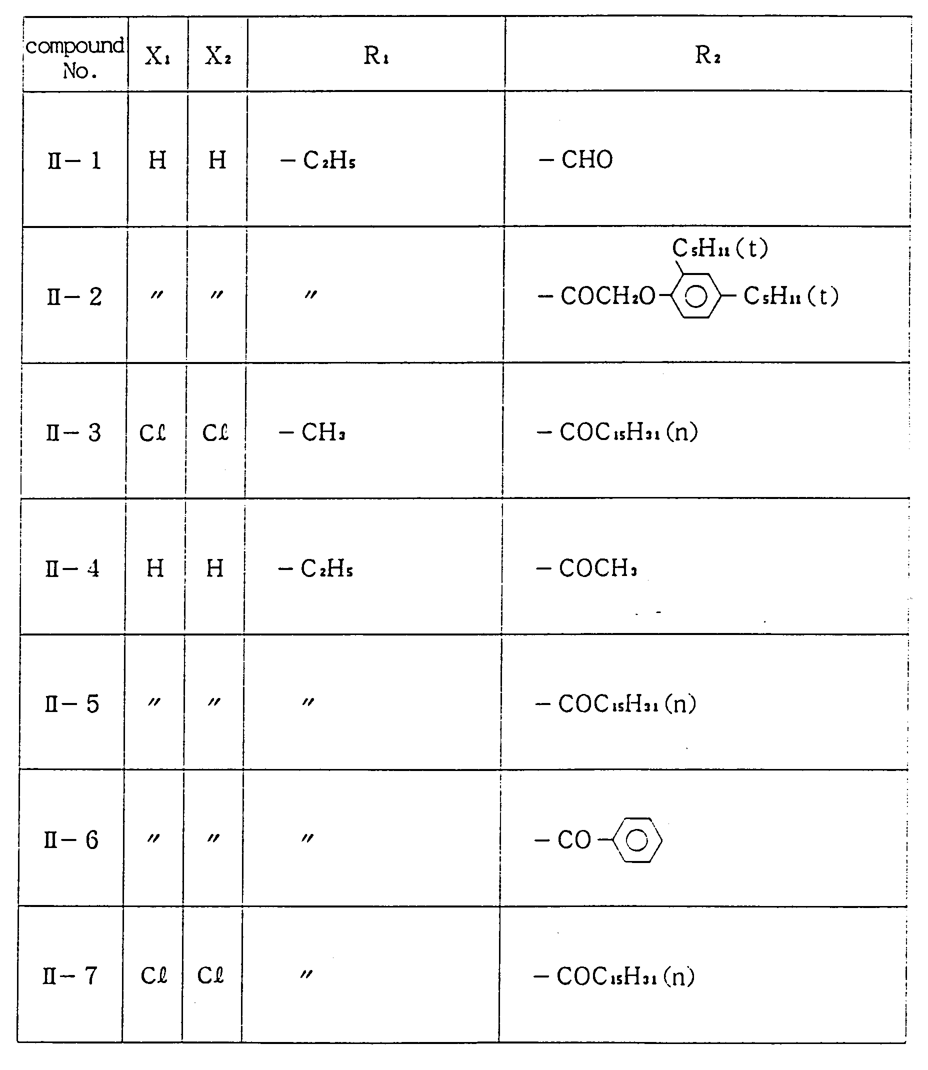

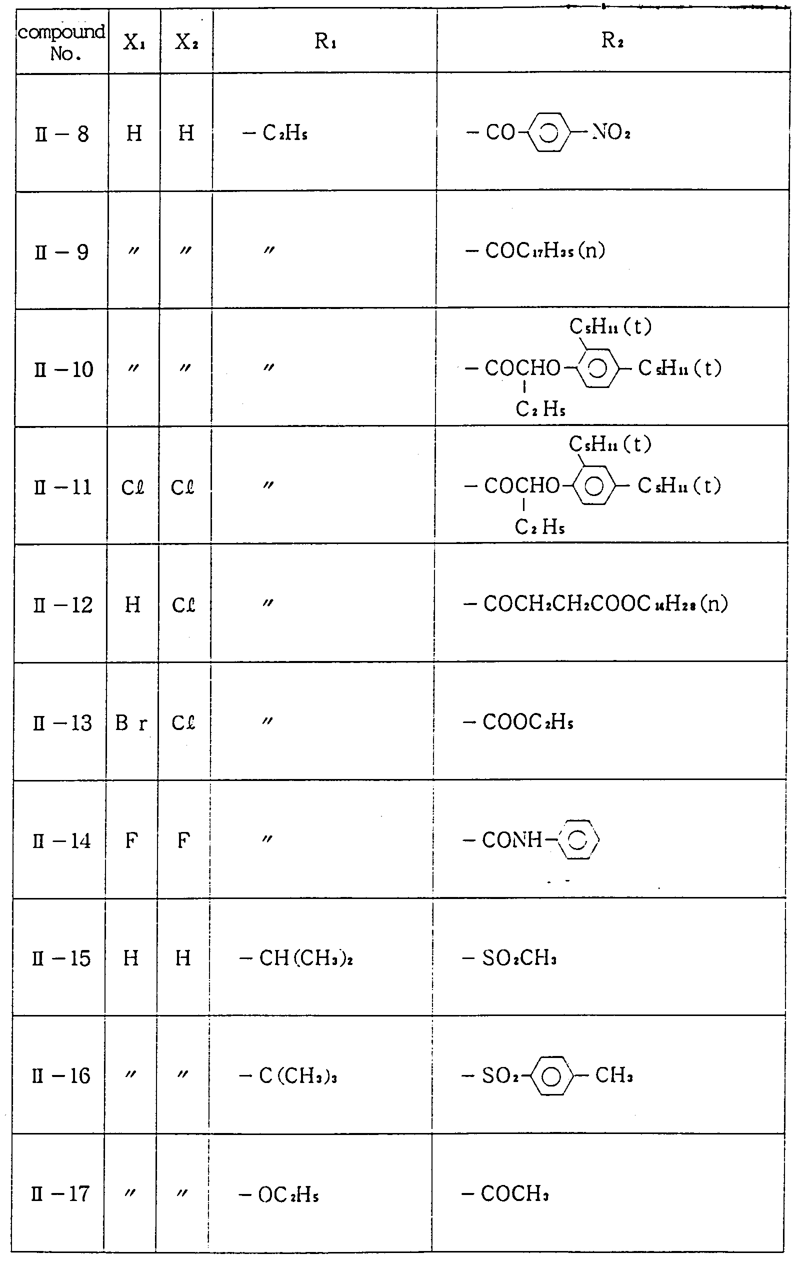

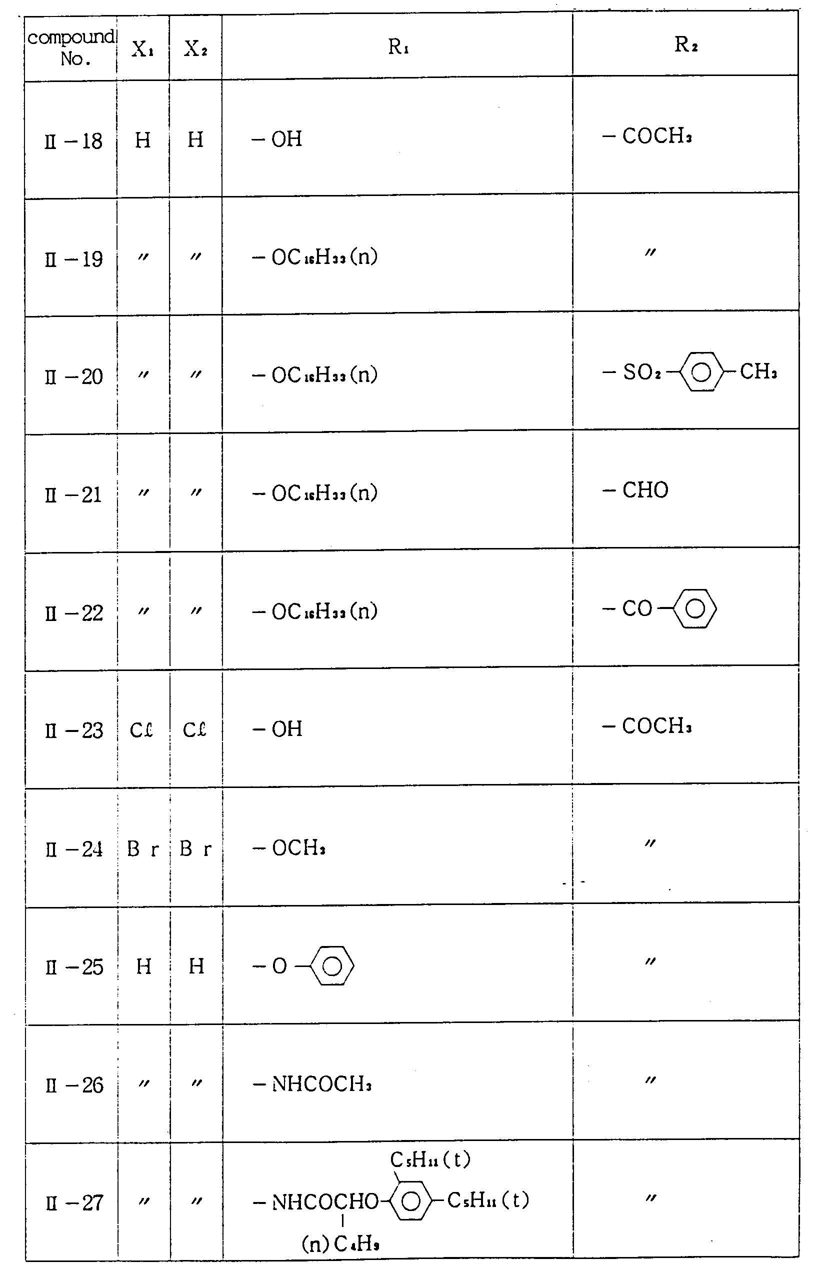

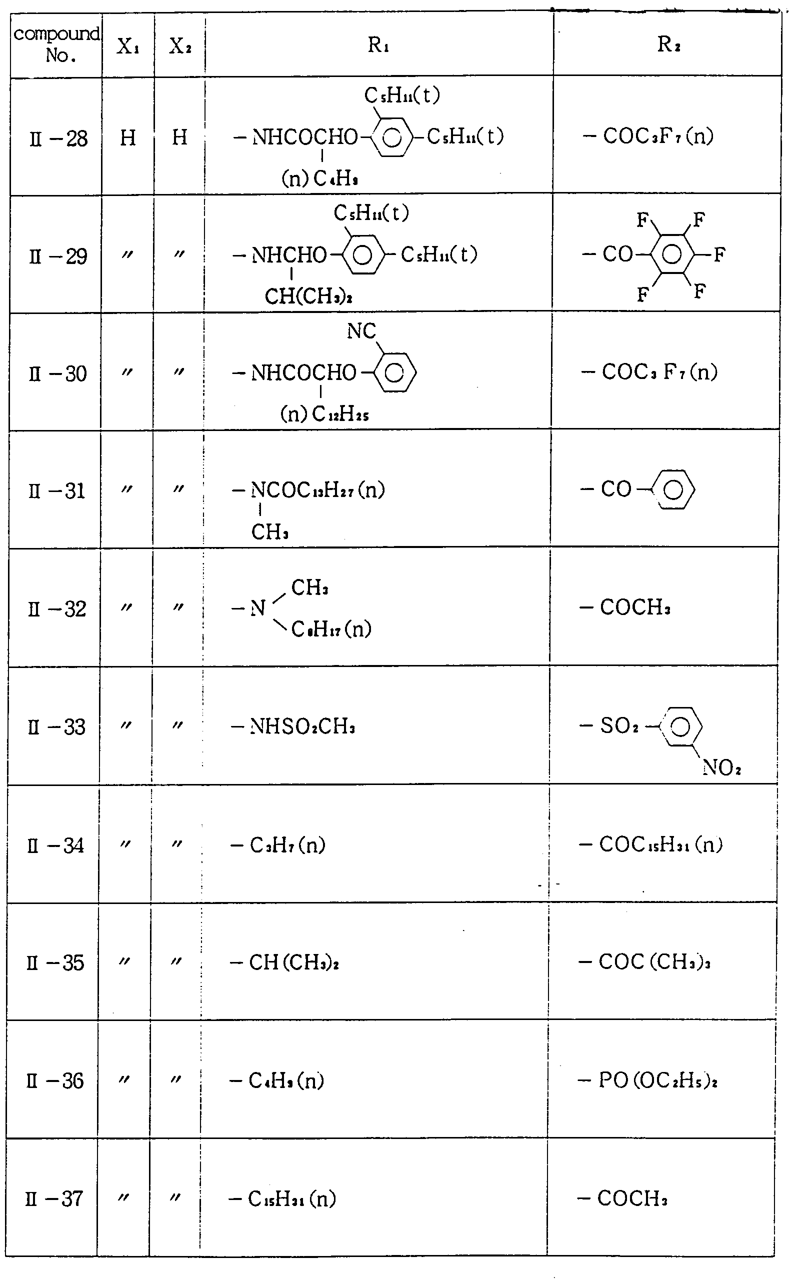

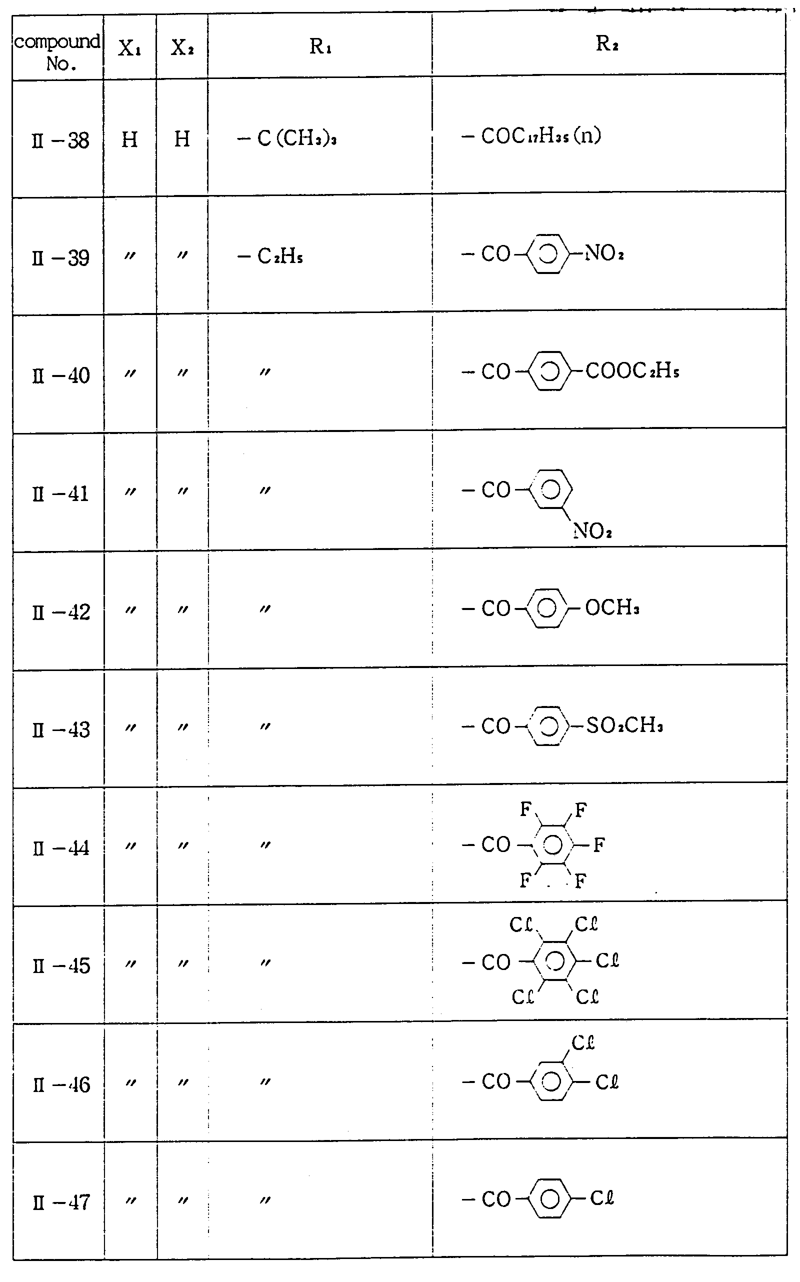

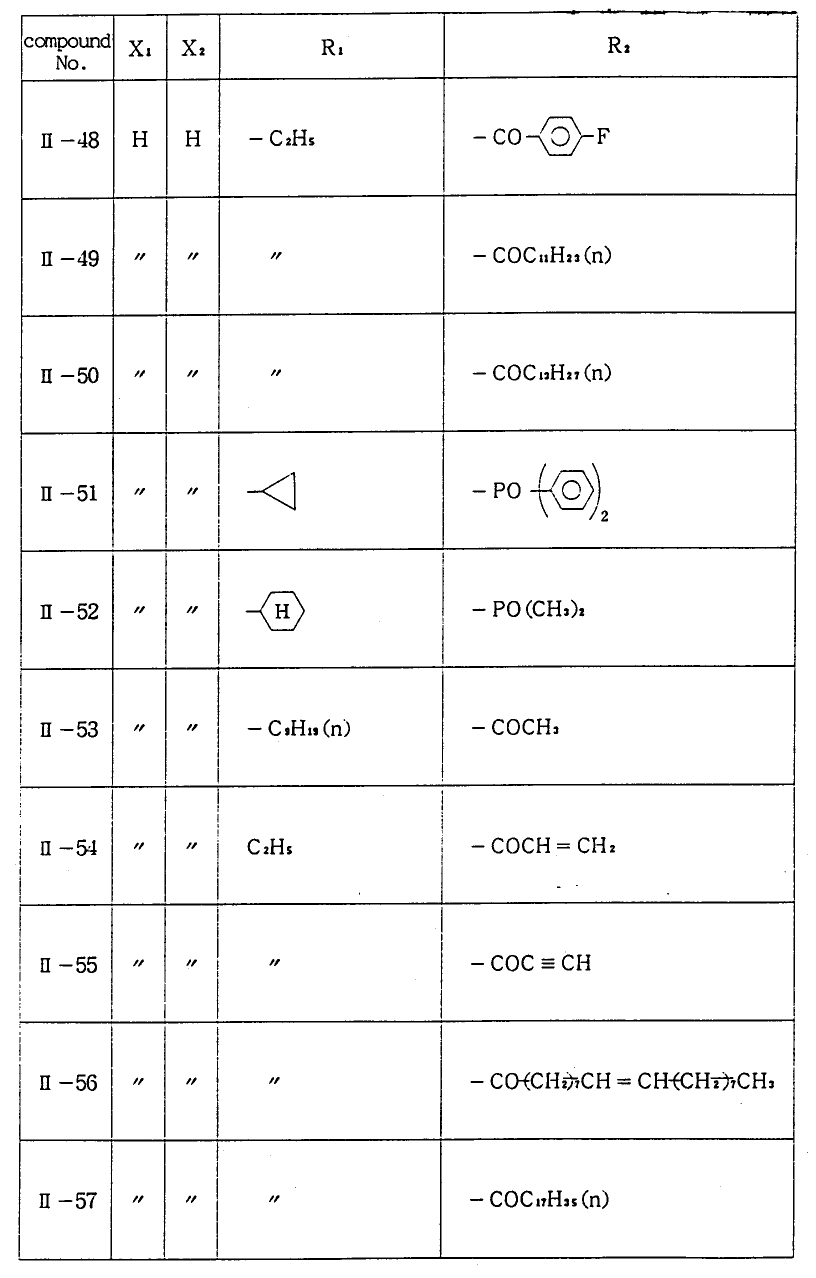

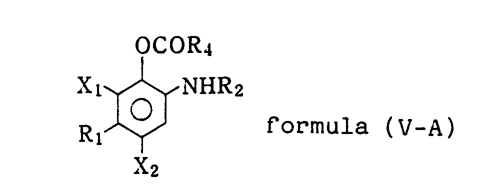

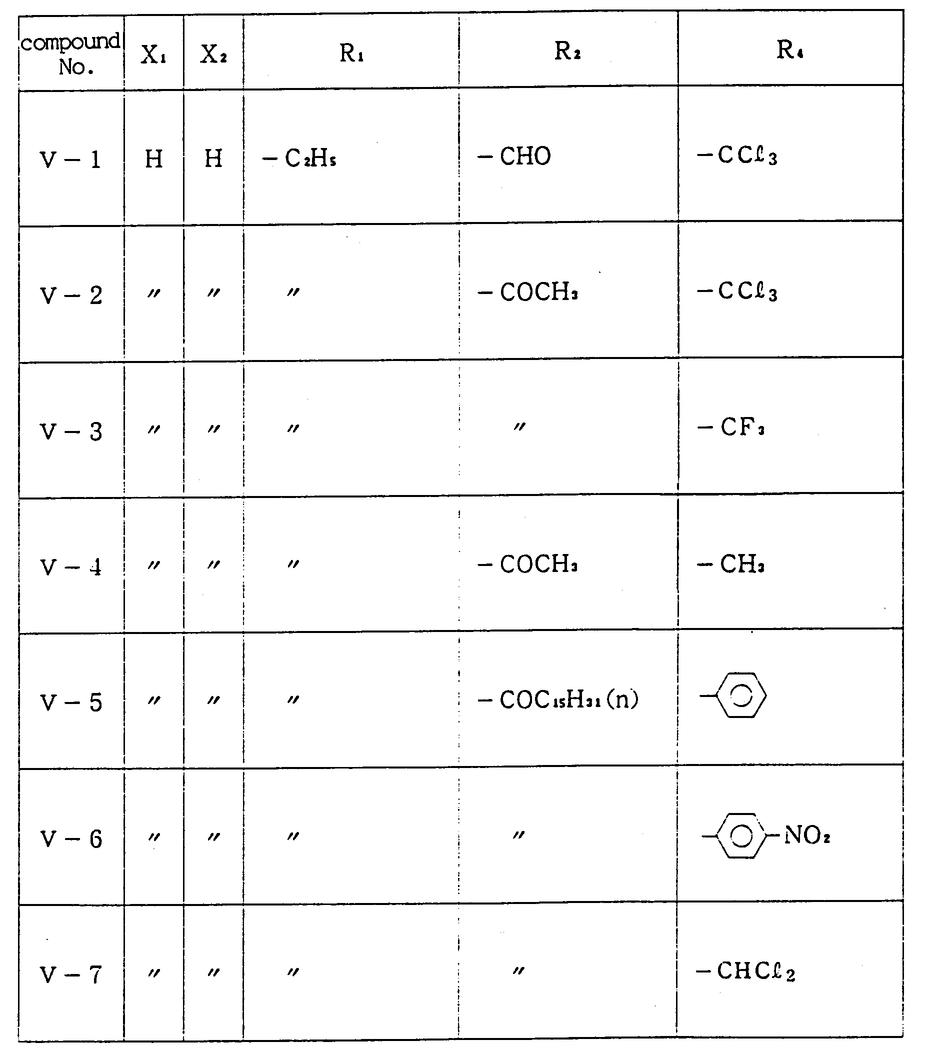

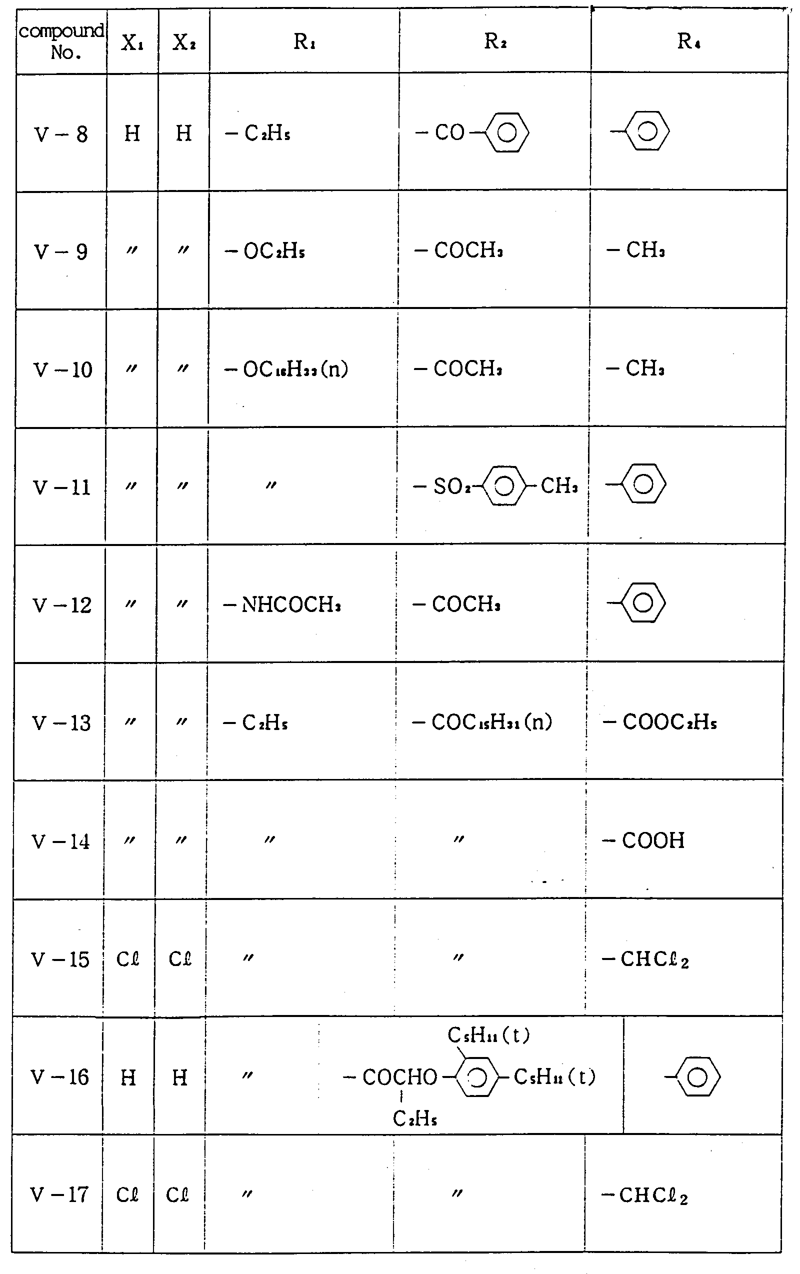

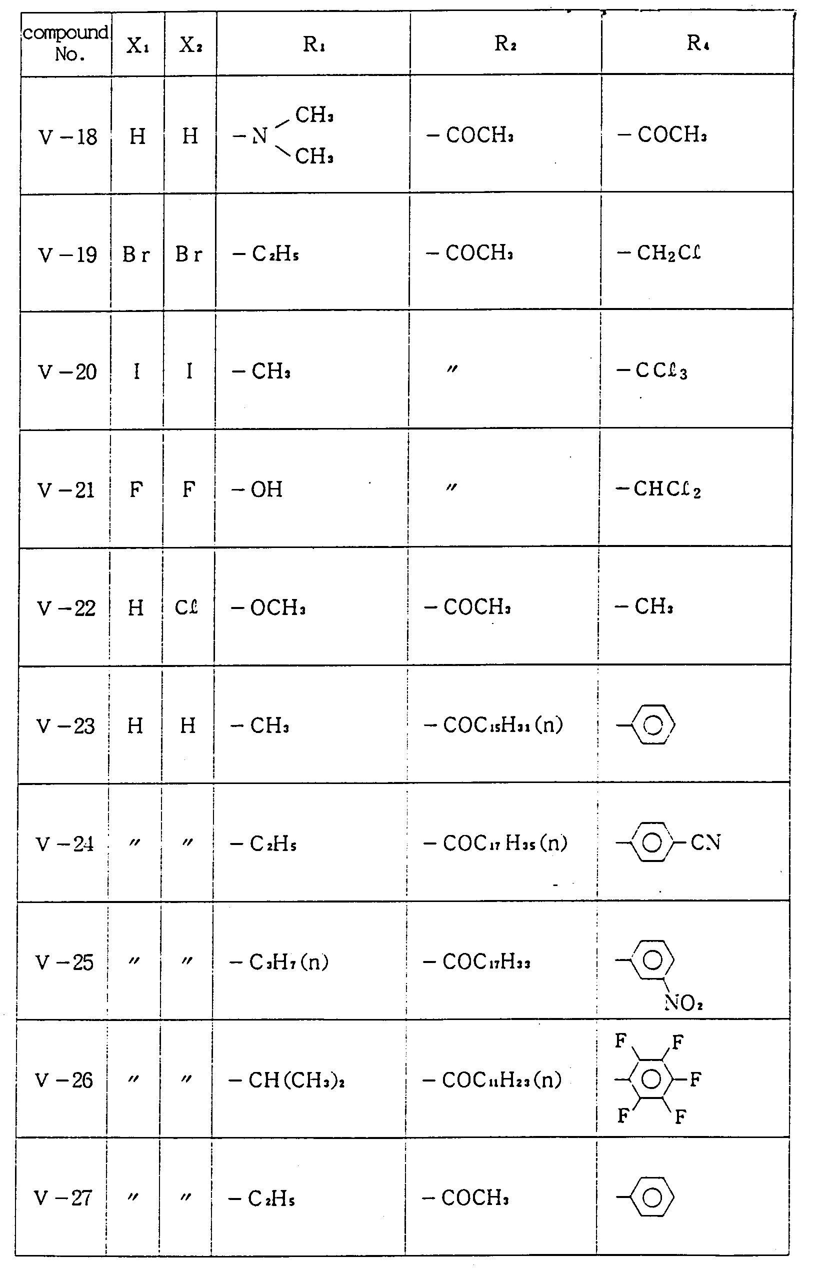

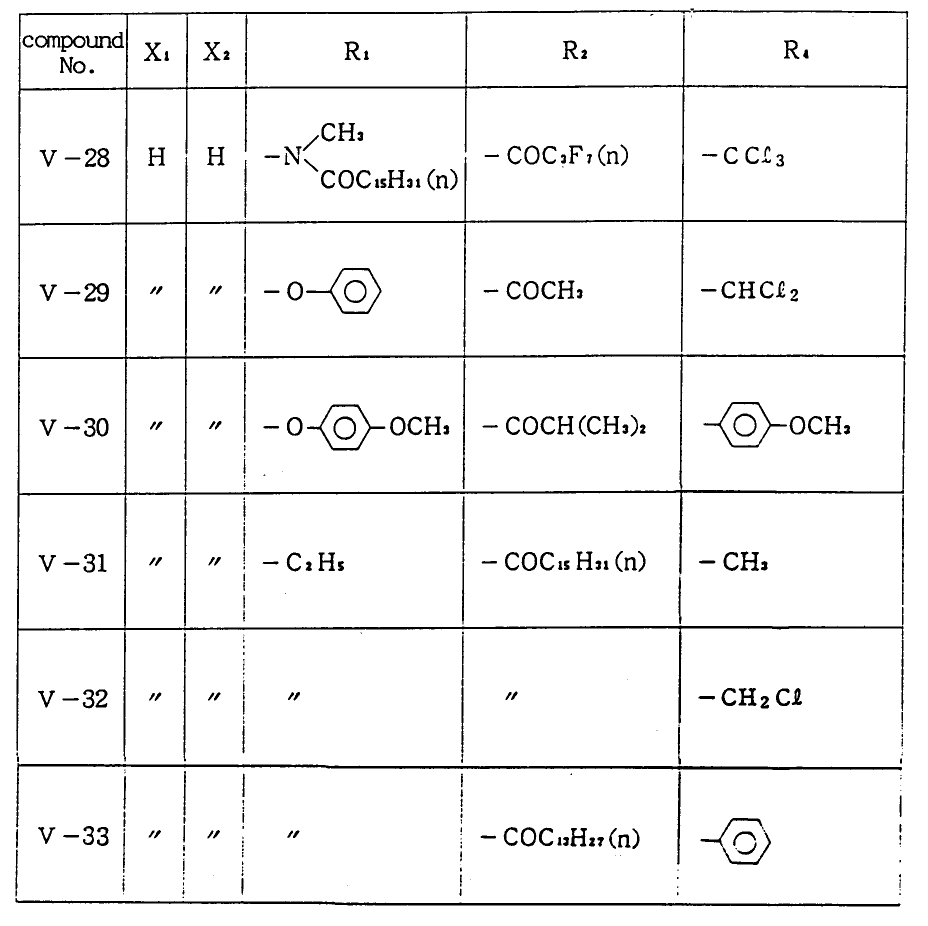

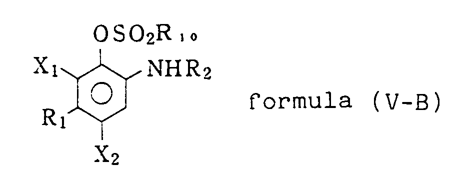

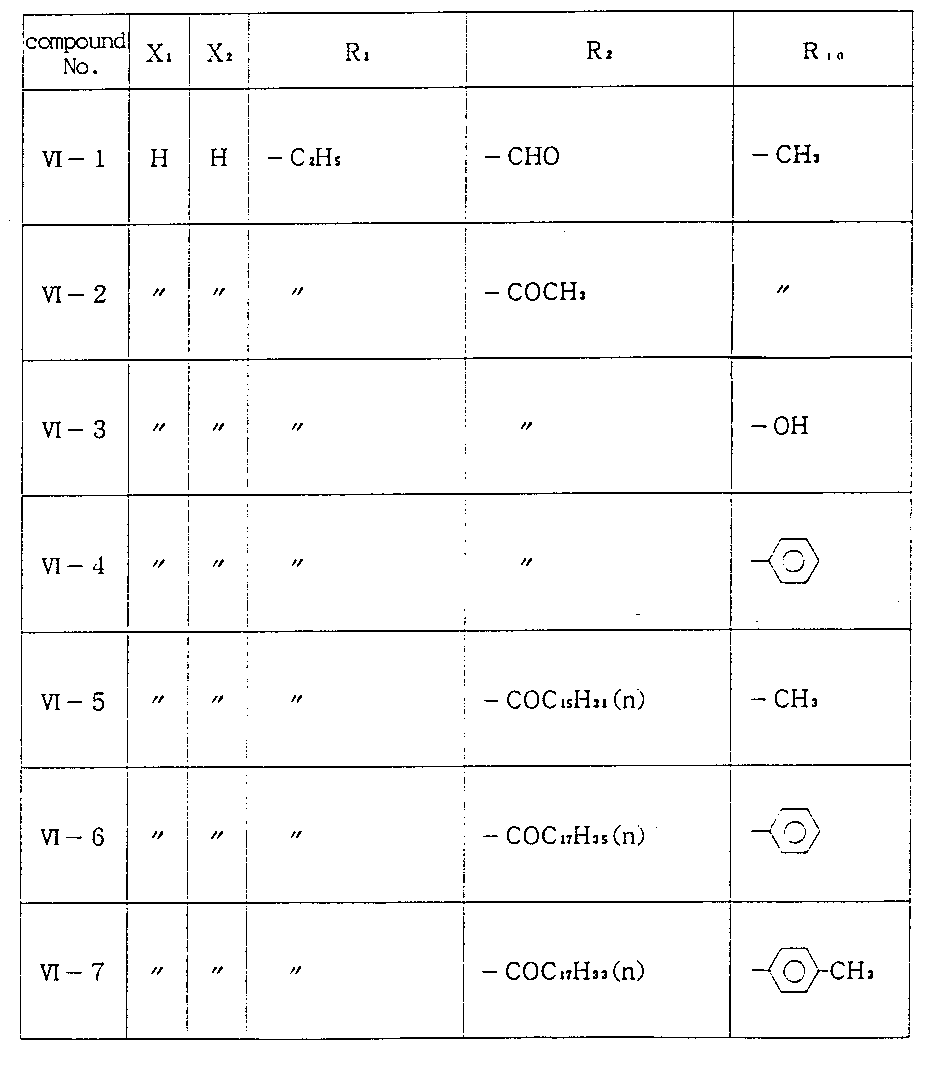

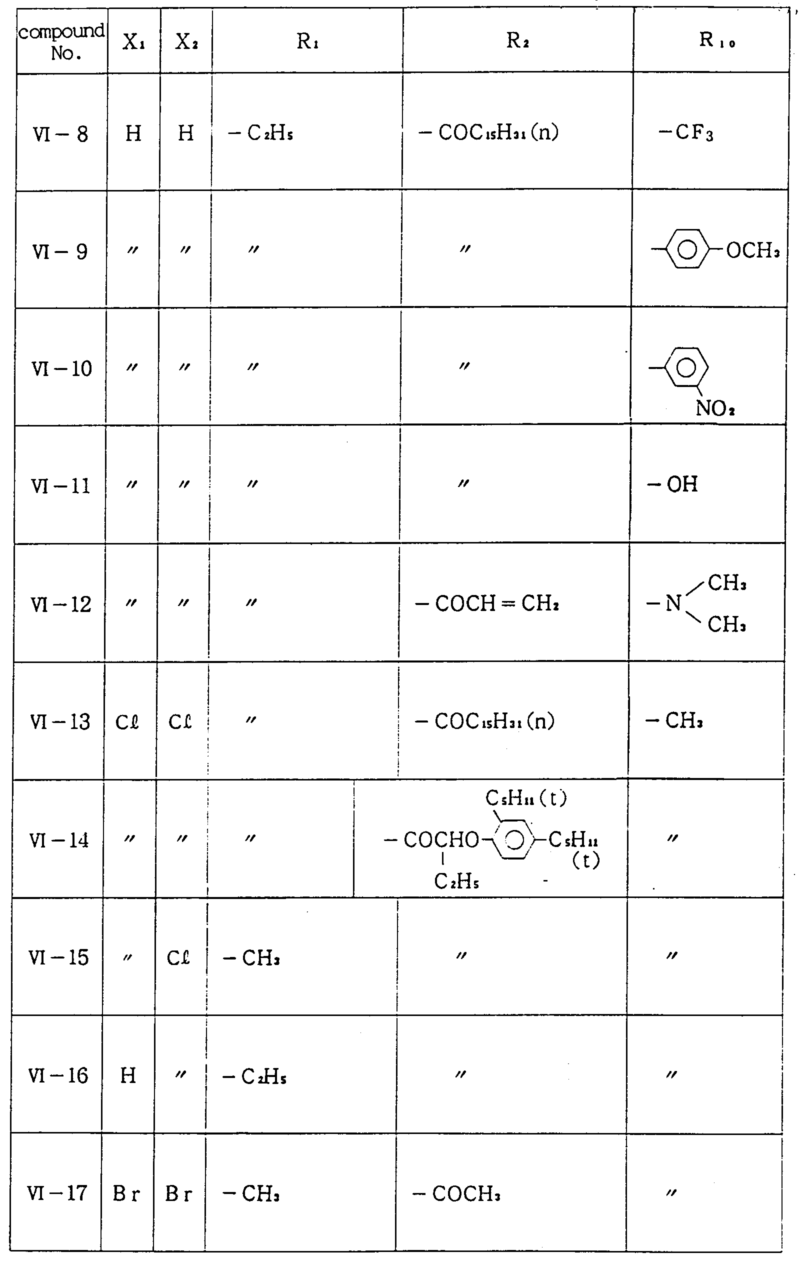

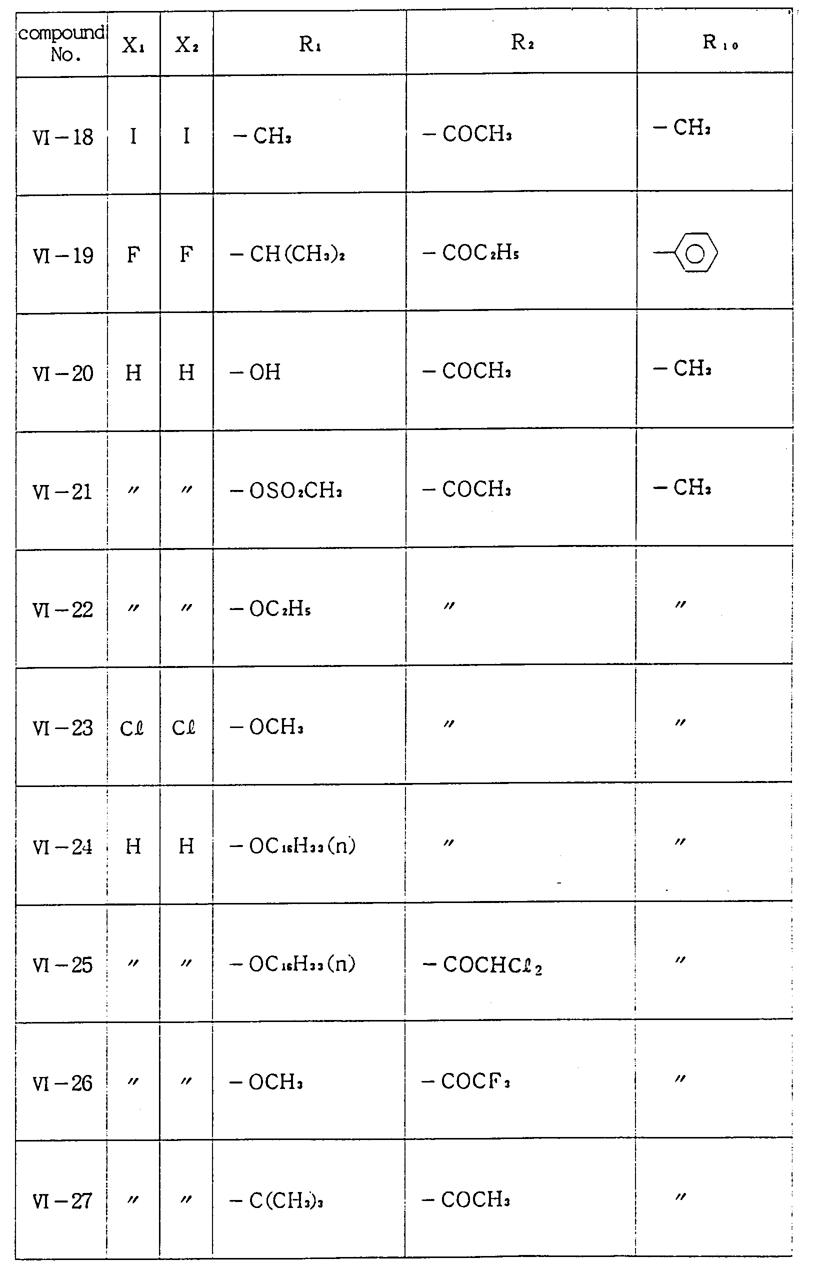

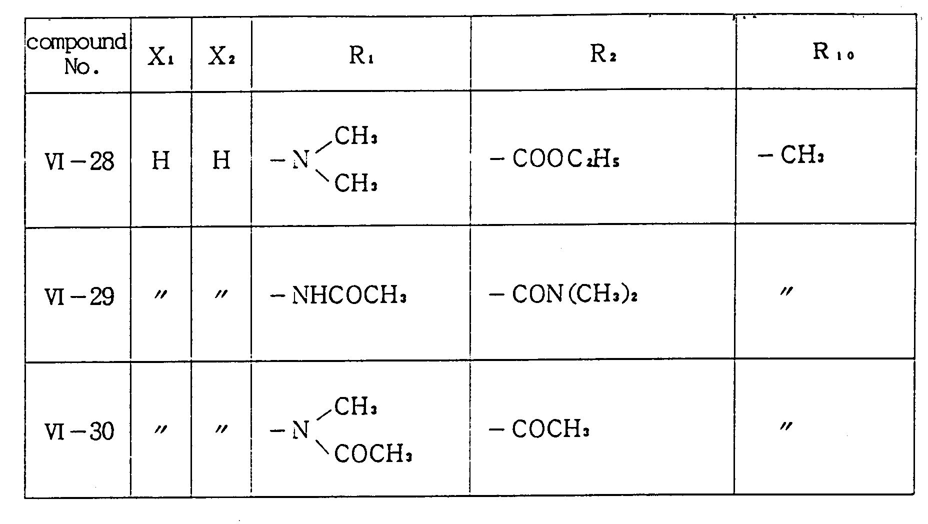

A scheme of the synthetic method according to the present invention is shown below: 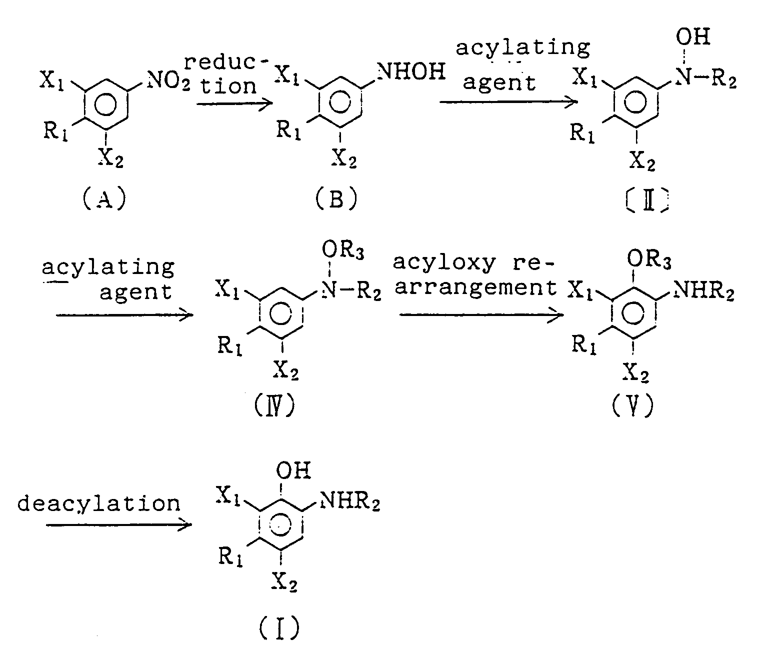 wherein R₁, R₂, R₃, X₁, and X₂ have the same meanings as defined above.

In this, the scheme for obtaining the compound represented by formula (I) from the compound represented by formula (II) is shown in detail below: 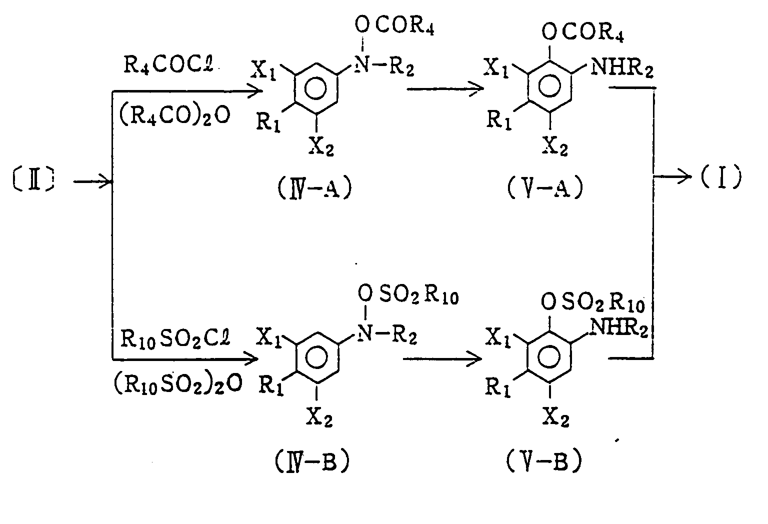 wherein R₁, R₂, R₄, R₅, X₁, and X₂ have the same meanings as defined above.

Now, the individual steps are described in detail:

The reduction reaction of the compound (A) to the compound (B) can be carried out in a usual manner, and it is generally carried out by using, for example, aluminum amalgam, catalytic reduction, electrolytic reduction, or zinc dust, as described in "Shin-Jikken Kagaku-Koza (Maruzen), Vol. 14, No. 3, 1978, page 1586.

Among them, the zinc dust reduction method and the catalytic reduction method are easy and effective. The former is carried out in accordance with the method described in Org. Synth., I, page 445 (1941), but if (A) is sparingly soluble in water-ethanol, the process has difficulty producing a good yield. With respect to the latter, since, in the reduction using hydrogen gas that is generally employed, the process of the reaction is hard to control (it is difficult to stop the process at the stage of the hydroxylamine without allowing reduction to the amine), it is preferable to use the method described in Synthesis, 1977, page 850 or ibid. 1984, page 938, which method is to use hydrazine as a source of hydrogen. In this method, since the solvent system is not restricted to water-ethanol, hydrophilic (A) is effectively reduced to (B).

To convert (B) to the compound represented by formula (II), a usual acylation reaction is used, and most of techniques for synthesizing amides are effective.

The acylating agent to be used is mainly the chloride and the anhydride of an acid corresponding to R₂ of formula (II), as well as acylating agents obtained from active acylating agents and carboxylic acids (which are, for example, used in synthesizing peptides) or mixed acid anhydrides resulting from a reaction, for example, with ethyl chlorocarbonate or methanesulfonyl chloride in the presence of the base in the reaction system. However, when R₂ is an N-alkylcarbamoyl or an N-arylcarbamoyl, an N-alkylisocyanate or an N-arylisocyanate is used. Specific examples are aliphatic carboxylic acid chlorides, such as acetyl chloride, palmitoyl chloride, acryloyl chloride, propiolyl chloride, oleyl chloride, 2-(2,4-di-t-amylphenoxy)butyroyl chloride, acryloyl chloride, pivaloyl chloride, 4-(4-nitrophenyl)butyroyl chloride, 3-tetradesiloxycarbonylpropionyl chloride, and perfluorobutyloyl chloride; aromatic carboxylic acid chlorides, such as benzoyl chloride, o-, m-, or p-nitrobenzoyl chloride, p-cyanobenzoyl chloride, 3,4-dichlorobenzoyl chloride, p-methoxybenzoyl chloride, m-acetylaminobenzoyl chloride, 1-naphthoyl chloride, 2-furoyl chloride, 2-thiophenylcarbonyl chloride, 3-pyridinecarbonyl chloride, and 4-pyridazinecarbonyl chloride; mixed acid anhydrides, such as a formic acid-methanesulfonic acid anhydride mixture and a perfluorobutyric acid-isopropyl chlorocarbonate anhydride mixture; alkyl chlorocarbonates, such as methyl chlorocarbonate, ethyl chlorocarbonate, and octyl chlorocarbonate; aryl chlorocarbonates, such as phenyl chlorocarbonate, 4-nitrophenyl chlorocarbonate, and 4-methoxyphenyl chlorocarbonate; N,N-disubstituted-carbamoyl chlorides, such as N,N-dimethylcarbamoyl chloride, N,N-diphenylcarbamoyl chloride, N-methyl-N-octylcarbamoyl chloride, or N-methyl-N-phenylcarbamoyl chloride; alkanesulfonyl chlorides, such as methanesulfonyl chloride, butanesulfonyl chloride, or dodecanesulfonyl chloride; arenesulfonyl chlorides, such as benzenesulfonyl chloride, p-toluenesulfonyl chloride, 1-naphthalenesulfonyl chloride, 3-nitrobenzenesulfonyl chloride, or 4-chloro-3-nitrobenzenesulfonyl chloride; phosphinoyl chlorides, such as dimethylphosphinoyl chloride, dibutylphosphinoyl chloride, diethoxyphosphinoyl chloride, bis(dimethylamino)phosphinoyl chloride, diphenylphosphinoyl chloride, or diphenoxyphosphinoyl chloride; or isocyanates, such as phenyl isocyanate, methyl isocyanate, n-octyl isocyanate, p-octyloxyphenyl isocyanate, p-nitrophenyl isocyanate, or m-nitrophenyl isocyanate.

Suitably the acylating agent is used in an amount of 0.9 to 1.5 equivalents, and preferably 1.0 to 1.2 equivalents, for the compound of general formula (B).

When the acylating agent is an aliphatic carboxylic acid chloride, the Schotten-Baumann method is effective, wherein the acylating reaction is carried out in a water-organic solvent using an inorganic base, such as sodium carbonate. If other acylating agents are used, the acylation can be carried out by the usual method, wherein the acylation is performed in an organic solvent using an organic base, such as a pyridine-type base and a trialkylamine-type base.

When an inorganic base is used, suitably the inorganic base is used in an amount of 2 to 4 equivalents, and preferably 2 to 3 equivalents, for the compound of formula (B); while when an organic base is used, it may be suitable that the organic base is used in an amount of 0.9 to 1.5 equivalents, and preferably 1.0 to 1.2 equivalents, for the compound of formula (B).

There are two methods for converting the compound of formula (II) to the compound of formula (I): one takes a course through formulae (IV-A) and (V-A), and the other takes a course through formulae (IV-B) and (V-B). The appropriate method is chosen in accordance with the purpose.

### 1) The method taking a course of formulae (IV-A) and (V-A)

The synthesis of the compound of formula (IV-A) is carried out by reacting of the compound of formula (II) with an acylating agent. The acylating agent to be used is, for example, an acid chloride, an acid anhydride, or a mixed acid anhydride. Specific example acylating agents are a carboxylic acid chloride, such as acetyl chloride, chloroacethyl chloride, dichloroacetyl chloride, trichloroacetyl chloride, oxalyl chloride, benzoyl chloride, p-nitrobenzoyl chloride, m-nitrobenzoyl chloride, m-methoxybenzoyl chloride, p-chlorobenzoyl chloride, 3,5-dichlorobenzoyl chloride, p-fluorobenzoyl chloride, and m-cyanobenzoyl chloride; a carboxylic acid anhydride, such as acetic anhydride, trifluoroacetic anhydride, benzoic anhydride, chloroacetic anhydride, dichloroacetic anhydride, and trichloroacetic anhydride; and a mixed acid anhydride prepared, for example, from a carboxylic acid, such as acetic acid, chloroacetic acid, dichloroacetic acid, and benzoic acid, and methanesulfonyl chloride, benzenesulfonyl chloride, or p-toluenesulfonyl chloride.

Suitably the acylating agent is used in an amount of 0.9 to 1.5 equivalents, and preferably 1.0 to 1.2 equivalents, for the compound of formula (II).

When the compound of formula (II) is reacted with the acylating agent, if the acylating agent is high in reactivity, it is not required to use a base, whereas if the acylating agent is low in reactivity, it is preferable to use a base. The base to be used is an organic base or an inorganic base. The organic base includes, for example, a pyridine-type base or a trialkylamine-type base, and the inorganic base includes, for example, a metal carbonate-type base or a metal hydroxide-type base. Preferably, pyridine, triethylamine, sodium carbonate, potassium carbonate, or sodium hydrogencabonate is used. It may be suitable that, when an inorganic base is used, it is used in an amount of 2 to 4 equivalents, and preferably 2 to 3 equivalents, for the compound of formula (II); whereas when an organic base is used, it is used in an amount of 0.9 to 1.5 equivalents, and preferably 1.0 to 1.2 equivalents, for the compound of formula (II). When an inorganic base is used, preferably the reaction is carried out in a two-phase system of an organic solvent and water.

The organic solvent to be used is an aromatic hydrocarbon solvent, an ether solvent, an ester solvent, an amide solvent, a nitrile solvent, or a halogenated hydrocarbon solvent; preferably it is an aromatic hydrocarbon solvent, an ester solvent, or a nitrile solvent; and particularly preferably it is toluene, ethyl acetate, or acetonitrile.

The easiness of the rearrangement reaction of the compound of formula (IV-A) to the compound of formula (V-A) varies depending on the type of R₄. When R₄ is a highly electron-attracting group (e.g., a trifluoromethyl, trichloromethyl, dichloromethyl, or polynitrophenyl group), the rearrangement reaction is quite easy, and since the reaction proceeds at room temperature or below, it is impossible to isolate (IV-A) at room temperature. In that case, it is suggested not to try to isolate (IV-A) but to convert it to (V-A) at a stroke. When R₄ is a less electron-attracting group (which definition is not distinct; e.g., a chloromethyl, or unsubstituted alkyl or phenyl group), the rearrangement reaction proceeds slowly, and it is possible to isolate (IV-A) at room temperature. In this case the rearrangement reaction temperature is suitably chosen and is generally 50 to 200 °C, and preferably 60 to 150 °C.

If the reaction proceeds slowly, since the presence of an acid catalyst can promote the reaction, the acid catalyst is effective for lowering the reaction temperature. The acid catalyst to be used is a carboxylic acid, a sulfonic acid, or a typical or transition metal Lewis acid, and more particularly a carboxylic acid, such acetic acid, benzoic acid, oxalic acid, or terephthalic acid; a sulfonic acid, such as methanesulfonic acid or benzenesulfonic acid; or a Lewis acid, such as BF₃·(C₂H₅)₂O, BCl₃, AlCl₃, ZnCl₂, ZnI₂, TiCl₄, or Ti(i-PrO)₄ (titanium tetraisopropoxide). Preferably the acid catalyst to be used is a carboxylic acid or a Lewis acid.

The amount of the catalyst to be used is suitably within the range of from 0.05 to 2.0 equivalents, and preferably from 0.1 to 1.0 equivalent for (IV-A).

The solvent used in the rearrangement reaction is an organic solvent, including an aromatic hydrocarbon solvent, an ether solvent, an ester solvent, an amide solvent, a nitrile solvent, a halogenated hydrocarbon solvent, and an alcohol solvent, or water. Particularly the organic solvent includes an aromatic hydrocarbon, such as toluene, benzene, ethylbenzene, xylene, or nitrobenzene; an ether, such as ethyl ether, butyl ether, tetrahydrofuran, or dioxane; an ester, such as ethyl acetate, butyl acetate, or ethyl propionate; an amide, such as formamide, acetamide, or N-methylpyrrolidone; a nitrile, such as acetonitrile or propionitrile; a halogenated hydrocarbon, such as dichloromethane, chloroform, or dichloroethane; and an alcohol, such as methanol, ethanol, isopropanol, butanol, ethylene glycol, or octanol.

When a high rearrangement reaction temperature (100 °C or over) is required, a preferable organic solvent is an aromatic hydrocarbon solvent, whereas when a low rearrangement reaction temperature is required, a preferable organic solvent is an ether solvent, an ester solvent, a nitrile solvent, or a halogenated hydrocarbon solvent. A particularly preferable organic solvent is toluene, tetrahydrofuran, ethyl acetate, acetonitrile, or dichloromethane.

The conversion (deacylation) from the compound of formula (V-A) to the compound of formula (I) can be carried out by usual alkali hydrolysis described below. However, when -OCOR₄ is hard to undergo usual alkali hydrolysis, the use of a strong alkali causes an acyl group exchange, and a compound wherein R₂ is replaced with COR₄ is concomitantly produced. To prevent this from occurring, the hydrolysis is carried out at a suitable pH (about 8 to 9), or deacylation is carried out by using a compound low in basicity but high in nucleophilicity. Such a compound is hydroxylamine or hydrazine hydrate, with preference given to hydroxylamine, which is used in an amount of 0.8 to 3.0 equivalents, and preferably 1.0 to 2.0 equivalents. The reaction temperature is -50 to 100 °C, and preferably -20 to 50 °C. The obtainable compound of formula (I) is generally good in crystallinity and is easily purified in many cases. One of the above methods is used to convert the compound of formula (V-A) to formula (I), and if crystals can be deposited only by rendering the reaction liquid acidic, the purification is possible by filtration; whereas if the crystallization takes place rather poorly, first, extraction is carried out, and then the purification is carried out by crystallization. If the crystallization takes place poorly, it is needless to say that purification by a column chromatography or a usually used method can be used to isolate the product.

### 2) The method taking a course of formulae (IV-B) and (V-B)

The synthesis of compound of formula (IV-B) is carried out by reacting the compound of formula (II) with an acylating agent (sulfonylating agent). The sulfonylating agent to be used is, for example, a sulfonyl chloride, a sulfonic acid anhydride, or sulfuric acid anhydride (SO₃). Besides sulfonic acid anhydride, example sulfonylating agents are a sulfonyl chloride, such as methanesulfonyl chloride, benzenesulfonyl chloride, p-toluenesulfonyl chloride, p-chlorobenzenesulfonyl chloride, p-nitrobenzenesulfonyl chloride, m-nitrobenzenesulfonyl chloride, and 4-chloro-3-nitrobenzenesulfonyl chloride; and a sulfonic acid anhydride, such as methanesulfonic acid anhydride, benzenesulfonic acid anhydride, p-toluenesulfonic acid anhydride, and trifluoromethanesulfonic acid anhydride. Suitably the sulfonylating agent is used in an amount of 0.9 to 1.5 equivalents, and preferably 1.0 to 1.2 equivalents, for the compound of formula (II).

When the compound of formula (II) is reacted with the sulfonylating agent, generally a base is used, which is an organic base. Example bases are a pyridine base, such as pyridine, lutidine, and collidine; a trisubstituted amine base, such as triethylamine, tributylamine, dimethylaniline, diethylaniline, or 1,4-diazabicyclo[2.2.2]octane (DABCO); or a bicyclic amidine base, such as 1,5-diazabicyclo[4.3.0]-5-nonene (DBN) or 1,8-diazabicyclo[5.4.0]-7-undecene (DBU). Preferably a pyridine base or a trialkylamine base is used, and particularly preferably pyridine or triethylamine is used. Suitably these bases are used in an amount of 0.9 to 1.5 equivalents, and preferably 1.0 to 1.2 equivalents, for the compound of formula (II).

The reaction solvent to be used is an aromatic hydrocarbon solvent, an ether solvent, an ester solvent, an amide solvent, a nitrile solvent, or a halogenated hydrocarbon solvent; preferably it is an aromatic hydrocarbon solvent, an ether solvent, an ester solvent, or a nitrile solvent; and particularly preferably it is toluene, tetrahydrofuran, ethyl acetate, acetonitrile, or dichloromethane.

The reaction temperature of the sulfonylation is -78 to 30 °C, and preferably -78 to 10 °C. Since the compound of formula (IV-B) that has been sulfonylated quite easily undergoes rearrangement to be changed to the compound of formula (V-B), it is quite difficult to isolate the compound of formula (IV-B) at room temperature. Therefore, conversion from the compound of formula (II) to the compound of formula (V-B) is generally carried out without isolating the compound of formula (IV-B); that is, along with the sulfonilation, the reaction proceeds automatically to the compound of formula (V-B).

The conversion (deacylation) from the compound of formula (V-B) to the compound of formula (I) is generally carried out by alkali hydrolysis, and an acyl group exchange, as in the case of the conversion from the compound of formula (V-A) to the compound of formula (I), is not observed.

The alkali hydrolysis is carried out by adding an aqueous solution of a hydroxide of an alkali metal or an alkali earth metal, such as lithium hydroxide, sodium hydroxide, potassium hydroxide, or barium hydroxide, to the compound of formula (V-A) or formula (V-B) dissolved in an organic solvent. It may be suitable to use such a hydroxide of an alkali metal or an alkali earth metal in an amount of 1 to 30 equivalents, and preferably 1 to 5 equivalents, for the compound represented by formula (V-A) or (V-B). To make the reaction liquid uniform, a water-soluble organic solvent, such as an alcohol solvent, is suitably added, followed by stirring. Since the reaction speed is not very high at room temperature, heating is generally performed, to promote the reaction. The reaction temperature is 20 to 120 °C, and preferably 25 to 50 °C.

After the reaction, the reaction liquid is made acidic from neutral, and the compound of formula (I) is isolated and purified, for example, by crystallization, extraction, or column chromatography.

In the compound of formula (I) synthesized by the above two methods, when both or one of X₁ and X₂ is a hydrogen atom, by reacting the compound with a halogenating agent, X₁ and X₂ both can be converted to halogen atoms. The halogenating agent to be used is chlorine, sulfuryl chloride, N-chlorosuccinimide, or the like in the case of chlorination, and bromine, N-bromosuccinimide, 1,3-dibromo-5,5-dimethylhydantoin, prydinium bromide perbromide, or the like in the case of bromination. Preferably the chlorination is carried out by using chlorine or sulfury chloride, and the bromination is carried out by using bromine.

As the reaction solvent, a halogen-type solvent, an amide-type solvent, an ester-type solvent, an organic acid-type solvent, an aromatic hydrocarbon-type solvent, an alcohol-type solvent, a ketone-type solvent, or an aqueous-type solvent, or a mixture of these, is used. Preferably an organic acid-type solvent or an aqueous-type solvent is used, and in the case of an aqueous-type solvent, preferably an aqueous acid solution is used. If the compound resists being dissolved in these solvents, a co-solvent, selected from the above solvents, can be suitably added.

The reaction temperature is 0 to 100 °C, and preferably 10 to 50 °C. The reaction time varies depending on the reactivity of the halogenating agent used, but generally it is 10 min to 24 hours, and preferably 30 min to 4 hours. The amount of the halogenating agent is 1.95 equivalents or more, and preferably 2.0 to 3.0 equivalents, for the compound of formula (I), when two halogen atoms are to be introduced. When one halogen atom is to be introduced and both X₁ and X₂ are hydrogen atoms, the halogenating agent is used in an amount of 0.8 to 1.2 equivalents, and preferably 0.95 to 1.05 equivalents; whereas when only one of X₁ and X₂ is a hydrogen atom and only one halogen atom is to be introduced, the amount of the halogenating agent is 0.95 to 2.0 equivalents, and preferably 1.1 to 1.5 equivalents.

According to the production method of the present invention, 2-acylaminophenol derivatives can be produced in short steps from inexpensively available nitrobenzene derivatives, which is an excellent function and effect. Further, the method of the present invention does not use substances that likely bring about contamination of the environment, and therefore it is high in safety. Thus, according to the present invention it has become possible to provide a cyan coupler for silver halide color photography and a dye-releasing agent for instant color photography inexpensively on an industrial scale.

Further, the compound of the present invention represented by formula (VIII) is a compound that does not appear in the literature; the compound represented by formula (VII), after chlorination, can be used as a cyan coupler for photography or as a compound for a photographic useful agent; and the compound represented by formula (VIII) can be used as a synthetic starting material of the compound represented by formula (VII), which is an excellent function and effect.

### EXAMPLE

Now, synthetic methods are described in detail with reference to the following examples.

### Example 1 (Synthesis of Exemplified Compounds (II-4) and (I-2))

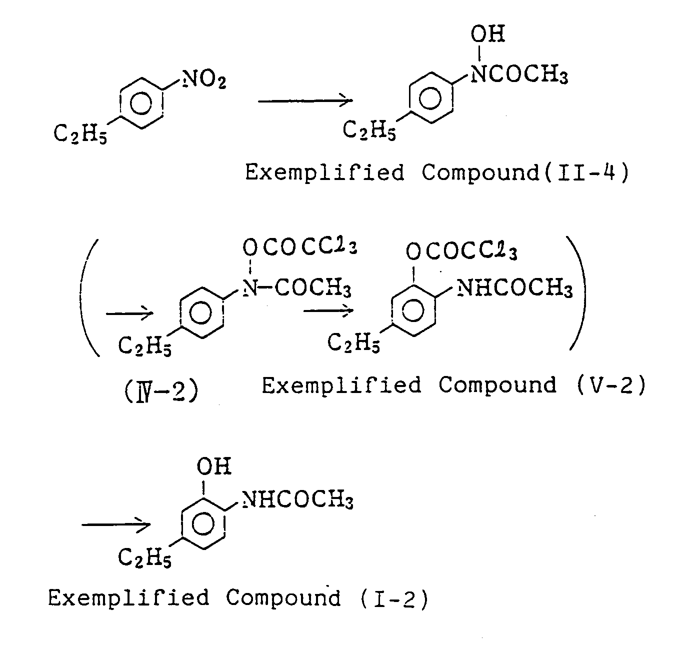

### 〈Synthesis of Exemplified Compound (II-4)〉

57.5 g (0.88 mol) of zinc was placed in a flask, and 100 ml of water was added thereto, followed by stirring. A solution of 60.4 g (0.4 mol) of 4-ethylnitrobenzene in 100 ml of ethanol was then added thereinto. While they were stirred vigorously under a stream of nitrogen, half of a solution of 25.6 g (0.48 mol) of ammonium chloride in 100 ml of water was added, all at once, through a dropping funnel thereinto. The internal temperature rose suddenly to 70 to 80 °C, and mild heating-up and reflux took place. After the reflux subsided a little, the rest of the solution was added, again all at once, followed by stirring. When the stirring was continued for about 1 hour, the internal temperature reached 20 to 30 °C, and then 200 ml of ethyl acetate was added, followed by stirring. Insoluble matter (zinc hydroxide) was filtered off using Celite (suction filtration) and washed with 100 ml of ethyl acetate. The filtrate was placed in a separating funnel, the organic layer (containing N-(4-ethylphenyl)hydroxylamine), obtained by removing the aqueous layer, was transferred into a flask, and an aqueous solution of 160 g (1.51 mol) of sodium carbonate in 300 ml of water was added thereto. The resulting mixture was cooled to about 5 °C with ice water, followed by stirring vigorously, and 34.0 ml (0.48 mol) of acetyl chloride was added, dropwise, thereinto over about 30 min (the Schotten-Baumann method). After stirring was continued for about 1 hour, the reaction liquid was placed in a separating funnel, and the aqueous layer was separated and then extracted once with 200 ml of ethyl acetate. The extract was combined with the organic layer, the combined liquid was washed with water once, and then with brine once; then it was dried over magnesium sulfate, filtered, and condensed under reduced pressure, to obtain 66 g of a reddish liquid. Since this liquid contained a small amount of 4-ethylacetoanilide, the liquid was purified by silica gel chromatography, thereby obtaining 57 g (79.5%) of Exemplified Compound (II-4) as a crystalline compound.

### NMR (CDCl₃) of Exemplified Compound (II-4)

- δ: 1.25 (3H, t, J = 7.0), 2.09 (3H, brs) 2.69 (3H, q, J = 7.0), 7.15 to 7.40 (4H) 8.80 (1H, br.)

### 〈Synthesis of Exemplified Compound (I-2)〉

### 1) The method that uses trichloroacetyl chloride

18 g (0.10 mol) of Exemplified Compound (II-4) was dissolved in acetonitrile, and 20.0 g (0.11 mol) of trichloroacetyl chloride was added to the solution at room temperature, followed by stirring for about 3 hours. The reaction was followed by TLC, and it was found that Compound (IV-2) was quite unstable and was easily rearranged to yield Compound (V-2). This Compound (V-2) could be isolated, but it was susceptible to hydrolysis. Therefore, the Compound (V-2) was not isolated but was subjected to alkali hydrolysis at a stroke (by the addition of an aqueous 2-N NaOH solution at room temperature with stirring); then the pH was brought to 3 or below by adding hydrochloric acid, and after a usual extraction operation with ethyl acetate was carried out, condensation under reduced pressure was carried out, to obtain a crude product of Exemplified Compound (I-2). The crude product was purified by a silica gel short column, thereby obtaining 16.2 g (90%) of pure Exemplified Compound (I-2) as crystals.

### NMR (CDCl₃) of Exemplified Compound (I-2)

- δ: 1.20 (3H, t, J = 7.0), 2.22 (3H, s), 2.57 (2H, q, J = 7.0) 6.65 (1H, dt, J = 7.0, 2.0), 6.82 (1H, s), 6.87 (1H, d, J = 7.0) 7.54 (1H, brs), to 9.0 (1H, br)

### 2) The method that uses benzoyl chloride/benzoic acid

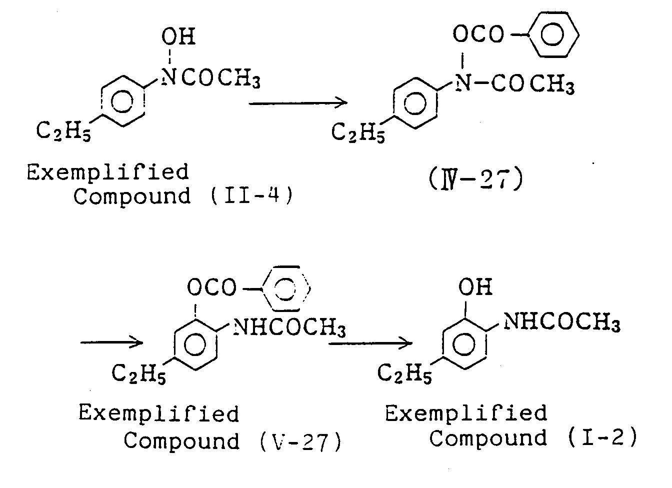

18 g (0.10 mol) of Exemplified Compound (II-4) was dissolved in 100 ml of toluene, and then 13.9 ml (0.10 mol) of triethylamine was added thereinto, at room temperature, followed by stirring. Then a solution of 14 g (0.10 mol) of benzoyl chloride in 20 ml of toluene was added, dropwise, thereinto, followed by stirring for about 30 min. Water was added to the reaction mixture, and an extraction operation with ethyl acetate was carried out, thereby obtaining 28 g (99%) of an oil of nearly pure Compound (IV-27).

### NMR (CDCl₃) of Compound (IV-27)

- δ: 1.22 (3H, t, J = 7.0), 2.12 (3H, brs) 2.66 (2H, q, J = 7.0), 7.24 (2H, d, J = 7) 7.4 to 7.5 (4H, m), 7.60 (1H, brt, J = 7) 8.08 (2H, d, J = 7.0)

To 28 g (0.099 mol) of this Compound (IV-27), was added 12 g (0.098 mol) of benzoic acid, followed by addition of 10 ml of toluene, and then the reaction mixture was heated, with stirring, on an oil bath having an external temperature of 120 °C, under a stream of nitrogen, and the reaction was allowed to continue for 4 hours, thereby obtaining Compound (V-27). Thereafter the temperature, of the reaction liquid was brought to room temperature, and 20 ml of toluene and 30 ml of methanol were added thereinto. Then, a 50% aqueous solution of 20 g (0.30 mol) of hydroxylamine was added, under cooling with ice, followed by stirring. About one hour later, the mixture was rendered acidic (pH 3 or below), an extraction operation with ethyl acetate was carried out, and the resulting product was purified by silica gel chromatography, thereby allowing 16.6 g (92%) of Exemplified Compound (I-2) to be obtained.

### Example 2 (Synthesis of Exemplified Compounds (II-5) and (I-5))

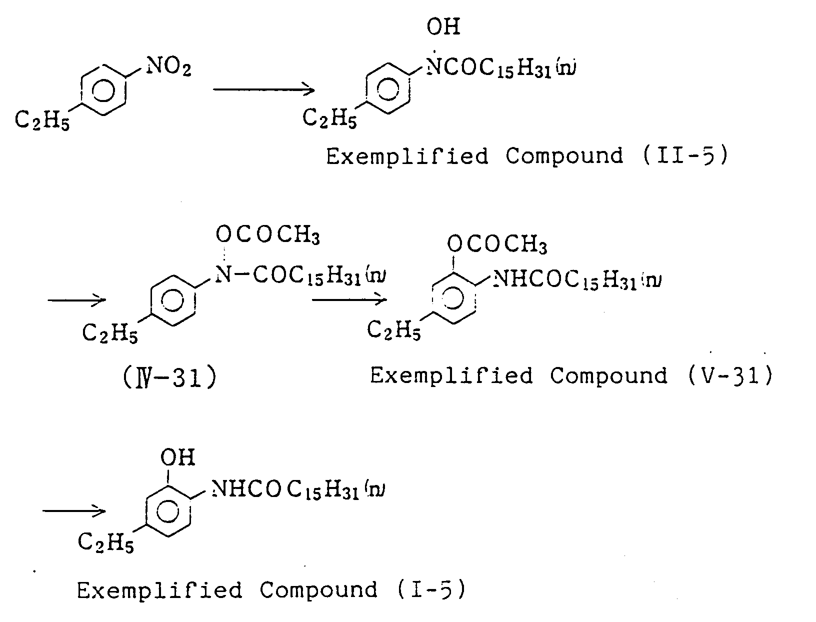

### 〈Synthesis of Exemplified Compound (II-5)〉

In a similar manner to Example 1, N-(4-ethylphenyl)hydroxylamine was synthesized, and, in the place of acetyl chloride, 110 g (0.40 mol) of palmitoyl chloride was used to carry out a similar reaction. Differently from Example 1, Exemplified Compound (II-5) crystallized out of the reaction liquid; the crystals were filtered under suction, washed with acetonitrile, and dried, to obtain 116.8 g (yield: 77.7%) of Exemplified Compound (II-5) as white crystals.

Melting point: 84 to 85 °C (recrystallized from ethyl acetate)

### NMR (CDCl₃) and IR (KBr) of Exemplified Compound (II-5)

- δ: 0.88 (3H, t, J = 7.0), 1.12 to 1.35 (27H) 1.63 (2H, brquintet, J = 7), 2.26 (2H, brt, J = 7) 2.69 (2H, q, J = 7.0), 7.2 to 7.33 (4H), 8.95 (1H, br)
- ν: 3200, 2930, 2860, 1640, 1510 cm⁻¹

### 〈Synthesis of Exemplified Compound (I-5) from Exemplified Compound (II-5)〉

### (1) The method that uses CH₃COCl/AlCl₃

7.4 ml (0.053 mol) of triethylamine was added to a solution of 20 g (0.053 mol) of Exemplified Compound (II-5) in 200 ml of ethyl acetate, followed by stirring at room temperature. Then, 3.8 ml (0.053 mol) of acetyl chloride was added, dropwise, thereinto. After about 30 min of stirring, an extraction operation with ethyl acetate was carried out, and the extract was purified by a silica gel column, to obtain 20 g (yield: 90%) of Compound (IV-31).

### NMR (CDCl₃) of Compound (IV-31)

- δ: 0.89 (3H, t, J = 7.0), 1.1 to 1.35 (27H) 1.62 (2H, brquintet, J = 7), 2.19 (3H, s) 2.20 (2H, br), 2.70 (2H, q, J = 7.0) 7.27 (2H, d, J = 7.0), 7.35 (2H, d, J = 7.0)

Then, 10 g (0.024 mol) of this Compound (IV-31) was dissolved in 100 ml of toluene, 1 g (0.0075 mol) of aluminum chloride was added to the solution, and the mixture was heated for about 10 min under reflux, to obtain Exemplified Compound (V-31). After the reaction liquid was cooled with ice water to about 5 °C, 100 ml of methanol was added thereto, and then an aqueous solution (about 50%) of 9.2 g (0.14 mol) of hydroxylamine was added, dropwise, thereto. After about 1 hour of stirring, the reaction mixture was rendered acidic, and an extraction operation with ethyl acetate was carried out. Thereafter the extract was purified by silica gel column chromatography, thereby enabling 5.4 g (yield: 60%) of Exemplified Compound (I-5) to be obtained.

Melting point: 72 to 73 °C (recrystallized from acetonitrile)

### NMR (CDCl₃) of Exemplified Compound (I-5)

- δ: 0.89 (3H, t, J = 7.0), 1.20 (3H, t, J = 7.0) 1.2 to 1.45 (24H), 1.75 (2H, quintet, J = 7.0) 2.43 (2H, t, J = 7.0), 2.59 (2H, q, J = 7.0) 6.69 (1H, dd, J = 8.0, 2.0), 6.89 (1H, d, J = 8.0) 6.88 (1H, d, J = 2.0), 7.40 (1H, brs) 8.88 (1H, s)

In this reaction, a by-product (α) was observed. The mass spectrum of (α) indicated that (α) had the same molecular weight as Exemplified Compound (I-5). From NMR it was found that (α) is a trisubstituted benzene having a hydroxyl group, an ethyl group, and a palmitoylamino group on the benzene ring. Thus, it is apparent that (α) is a compound wherein a hydroxyl group is rearranged to a position other than the ortho position.

### NMR (CDCl₃) of (α)

- δ: 0.89 (3H, t, J = 7.0), 1.20 (3H, t, J = 7.0) 1.2 to 1.4 (24H), 1.72 (2H, brquintet, J = 7) 2.36 (2H, t, J = 7.0), 2.60 (2H, q, J = 7.0) 6.50 (1H, dd, J = 7.0, 2.0), 7.02 (1H, d, J = 7.0) 7.11 (1H, brs), 7.30 (1H, brs) 7.78 (1H, d, J = 2.0)

### (2) The method that uses C₆H₅COCl/C₆H₅COOH

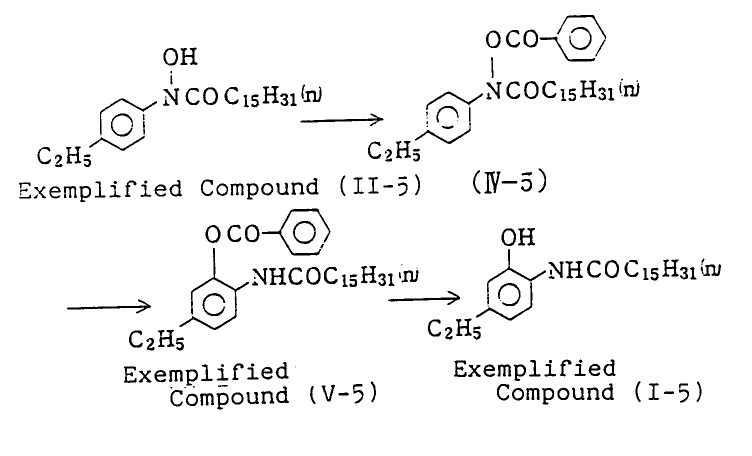

7.4 ml (0.053 mol) of triethylamine was added to a solution of 20 g (0.053 mol) of Exemplified Compound (II-5) in 20 ml of ethyl acetate, at room temperature, followed by stirring, and then a solution of 6.2 ml (0.053 mol) of benzoyl chloride in 5 ml of ethyl acetate was added, dropwise, thereto. After the addition, the stirring was continued at room temperature for about 1 hour; then water was added to the reaction mixture, and an extraction operation with ethyl acetate was carried out. An oil obtained by condensation of the extract was purified by silica gel chromatography, thereby obtaining 23.5 g (yield: 92.1%) of Compound (IV-5) as a colorless oil (upon allowing it to stand, it crystallized, but the melting point was low).

### NMR (CDCl₃) of Compound (IV-5)

- δ: 0.88 (3H, t, J = 7.0), 1.14 to 1.36 (27H) 1.70 (2H, brquintet, J = 7), 2.33 (2H, br) 2.70 (2H, q, J = 7.0), 7.28 (2H, d, J = 7) 7.4 to 7.5 (4H), 7.60 (1H, brt, J = 7) 8.10 (2H, d, J = 7.0)

23 g (0.048 mol) of Compound (IV-5) was placed into a flask; then 5.9 g (0.048 mol) of benzoic acid was placed in it, and 10 ml of toluene was added thereto. The resulting solution was heated for 4 hours in an oil bath having an external temperature of 120 °C, with stirring, thereby obtaining Exemplified Compound (V-5). After the temperature was brought to room temperature, 20 ml of toluene and 30 ml of methanol were added to the Compound (V-5), and a 50% aqueous solution containing 9.5 g (0.14 mol) of hydroxylamine was added, under cooling with ice, followed by stirring for about 1 hour. Thereafter, the pH was brought to about 3 using concentrated hydrochloric acid, and the toluene layer was separated and washed with water twice. The toluene layer was condensed under reduced pressure, and methanol was added to the concentrate, to cause crystallization, thereby enabling Exemplified Compound (I-5), in an amount of 16.2 g (yield: 90%), to be obtained.

In this reaction, when Compound (IV-5) was heated (to 120 °C) without using benzoic acid and any solvent, it took about 20 hours until the raw material disappeared, whereas when Compound (IV-5) was heated (to 120 °C) using benzoic acid in an amount of 0.1 equivalent, it took about 10 hours until the starting material disappeared.

In this reaction, it is possible that, after the reaction, the rearranged product obtained before the deacylating with a hydroxylamine, that is, Exemplified Compound (V-5), can be rid of the solvent and recrystallized from acetonitrile, to isolate it as colorless crystals.

### NMR (CDCl₃) of Exemplified Compound (V-5)

- δ: 0.89 (3H, t, J = 7.0), 1.1 to 1.35 (27H) 1.58 (2H, brquintet, J = 7), 2.26 (2H, t, J = 7.0) 2.65 (2H, q, J = 7.0), 7.05 (1H, s), 7.10 (1H, s) 7.12 (1H, d, J = 7.0), 7.53 (2H, t, J = 7.0) 7.68 (1H, t, J = 7.0), 7.97 (1H, d, J = 7.0) 8.21 (2H, d, J = 7.0)

### (3) The method that uses p-nitrobenzoyl chloride

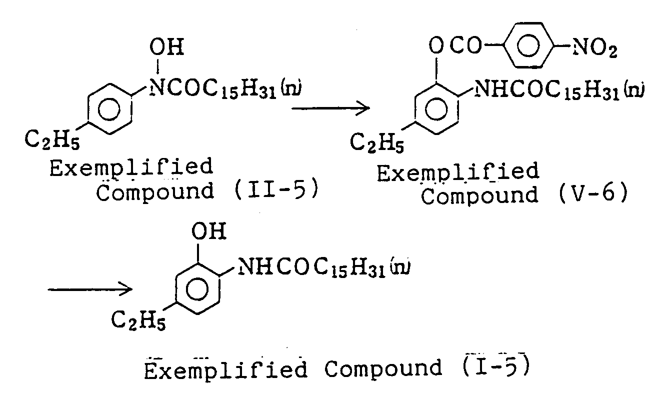

10.8 g (0.058 mol) of p-nitrobenzoyl chloride was added to a solution of 20 g (0.053 mol) of Exemplified Compound (II-5) in 100 ml of acetonitrile, followed by heating for about 8 hours under reflux. Upon bringing the temperature to room temperature, Exemplified Compound (V-6) deposited as crystals. The mixture was filtered and dried, to obtain 16 g of the crystals. Since the filtrate also contained Exemplified Compound (V-6), the filtrate was condensed and purified using a column, thereby enabling a further 6 g of Exemplified Compound (V-6) to be obtained.

Yield: 81%; Melting point: 116 to 117 °C

### NMR (CDCl₃) of Exemplified Compound (V-6)

- δ: 0.82 (3H, t, J = 7.0), 1.1 to 1.35 (27H) 1.59 (2H, brquintet, J = 7.0), 2.25 (2H, t, J = 7.0) 2.65 (2H, q, J = 7.0), 6.95 (1H, brs) 7.06 (1H, s), 7.15 (1H, d, J = 7.0) 7.82 (1H, d, J = 7.0), 8.40 (4H, s)

10 g (0.020 mol) of Exemplified Compound (V-6) was dissolved in 10 ml of toluene and 10 ml of methanol, and a 50% aqueous solution containing 2.6 g (0.039 mol) of hydroxylamine was added to the solution, at room temperature, followed by stirring for 1 hour. Thereafter, the same procedure as in the above (2) was carried out, so that 7.0 g (yield: 95%) of Exemplified Compound (I-5) could been obtained.

### (4) The method that uses chloroacetyl chloride

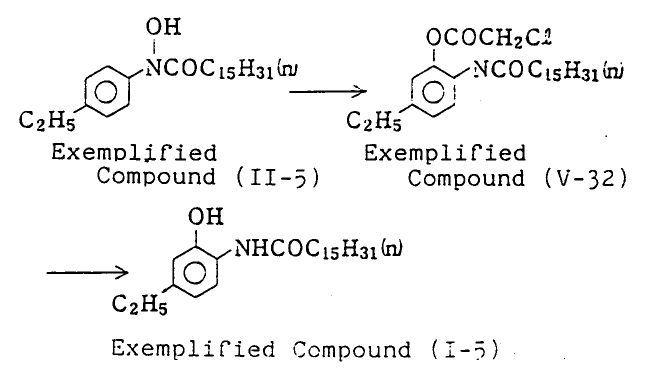

7.2 g (0.064 mol) of chloroacetyl chloride was added to a solution of 20 g (0.053 mol) of Exemplified Compound (II-5) in 100 ml of acetonitrile, and the mixture was heated for about 8 hours under reflux, thereby obtaining Exemplified Compound (V-32). The temperature was brought to room temperature, and in a similar manner to the above (2), deacylation with hydroxylamine and an isolation operation were carried out, thereby enabling 14.8 g (yield: 74%) of Exemplified Compound (I-5) to be obtained.

### (5) The method that uses dichloroacetyl chloride

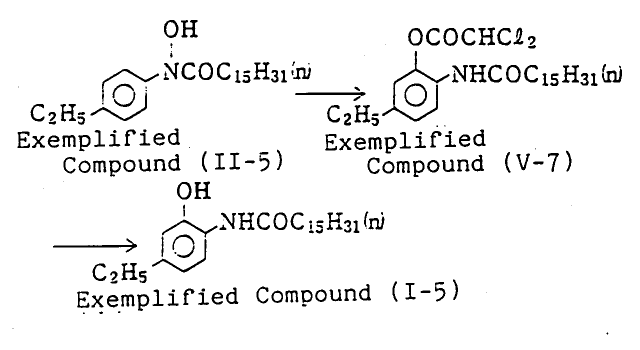

a) 9.6 g (0.064 mol) of dichloroacetyl chloride was added to a solution of 20 g (0.053 mol) of Exemplified Compound (II-5) in 100 ml of acetonitrile, and when the reaction mixture was stirred at room temperature for 3 hours, crystals deposited during the reaction. After allowing the reaction mixture to stand overnight, the deposited crystals were filtered and dried, thereby obtaining 22.4 g of Exemplified Compound (V-7).
   Yield: 87%; Melting point: 50 °C or below

### NMR (CDCl₃) of Exemplified Compound (V-7)

- δ: 0.89 (3H, t, J = 7.0), 1.23 (3H, t, J = 7.0) 1.2 to 1.4 (24H), 1.69 (2H, brquintet, J = 7) 2.32 (2H, t, J = 7.0), 2.62 (2H, q, J = 7.0) 6.23 (1H, s), 7.04 (1H, d, J = 2.0) 7.13 (1H, dd, J = 7.0, 2.0), 7.19 (1H, brs) 8.11 (1H, d, J = 7.0)

10 g (0.021 mol) of Exemplified Compound (V-7) was dissolved in a mixed solvent of 10 ml of toluene and 10 ml of methanol, and 10 ml (0.04 mol) of a 4-N sodium hydroxide aqueous solution was added to the solution, followed by stirring for about 1 hour. Thereafter the pH was brought to about 3 using hydrochloric acid, and in a similar manner to that of the above (2), an isolation operation was carried out, thereby enabling 7.3 g (yield: 95%) of Exemplified Compound (I-5) to be obtained.
b) 20 g (0.053 mol) of Exemplified Compound (II-5) was dispersed in 80 ml of toluene by stirring, and 4.8 ml (0.058 mol) of pyridine was added to the dispersion. The reaction liquid was cooled with water, and 5.6 ml (0.058 mol) of dichloroacetyl chloride was added, slowly, dropwise, to the reaction liquid. The reaction liquid turned pale yellow and pyridine hydrochloride deposited. The reaction mixture was placed on an oil bath and was heated for about 1 hour, to 100 °C. Then the temperature was brought to room temperature; then 20 ml of isopropanol and 10 ml of water were added to the reaction mixture, under cooling with water, and 25 ml (0.1 mol) of a 4-N sodium hydroxide solution was added thereto, followed by stirring for about 30 min. Thereafter, the pH was made to be about 3 with hydrochloric acid, and an isolation operation similar to the above was carried out, thereby enabling 17.0 g (yield: 85%) of Exemplified Compound (I-5) to be obtained.

### (6) The method that uses oxalyl chloride

5.2 ml (0.059 mol) of oxalyl chloride was added to a solution of 20 g (0.053 mol) of Exemplified Compound (II-5) in 100 ml of dichloromethane, at room temperature. The reaction mixture turned light yellow and HCl gas was released. After 15 min, 10 ml of methanol was added; the reaction mixture was stirred for about 30 min, and then 20 ml (0.08 mol) of a 4-N sodium hydroxide aqueous solution was added, followed by stirring for about 1 hour. Thereafter, the pH was brought to about 3 with hydrochloric acid, and an isolation operation similar to that of the above (2) was carried out, thereby enabling 15.9 g (yield: 80%) of Exemplified Compound (I-5) to be obtained.

### (7) The method that uses CH₃SO₂Cl

38 g (0.10 mol) of Exemplified Compound (II-5) was dissolved in 400 ml of dichloromethane, and then 16.7 ml (0.12 mol) of triethylamine was added to the solution. When a solution of 16.5 g (0.12 mol) of methanesulfonyl chloride in 5 ml of dichloromethane was added, dropwise, to the resulting mixture, under cooling with ice, heat was generated, and the reaction liquid turned light yellow. After the addition, the reaction liquid was stirred at about 10 °C for about 1 hour; then water was added thereto, and the dichloromethane layer was separated, washed with water, and then with brine; then it was dried over magnesium sulfate, followed by filtration. The filtrate was condensed under reduced pressure, thereby obtaining a crystalline residue. The crystalline residue was purified by silica gel column chromatography, thereby enabling 39 g (yield: 86%) of Exemplified Compound (VI-5) to be isolated.

### NMR (CDCl₃) of Exemplified Compound (VI-5)

- δ: 0.89 (3H, t, J = 7.0), 1.24 (3H, t, J = 7.0), 1.2 to 1.42 (24H), 1.70 (2H, brquintet, J = 7.0), 2.38 (2H, t, J = 7.0) 2.65 (2H, q, J = 7.0), 3.25 (3H, s), 7.08 (1H, d, J = 2.0), 7.15 (1H, dd, J = 7.0, 2.0), 7.75 (1H, brs), 8.10 (1H, d, J = 7.0)

20 g (0.044 mol) of Exemplified Compound (VI-5) was dissolved in a mixed solvent of 40 ml of tetrahydrofuran and 40 ml of methanol, and 45 ml (0.18 mol) of an aqueous 4-N sodium hydroxide solution was added to the resulting solution, followed by stirring at 40 °C for about 3 hours. Then water was added; the reaction mixture was made acidic (pH: 3) with concentrated hydrochloric acid, and an extraction operation with ethyl acetate was performed. After condensation of the extract, the obtained residue was purified by using a column, thereby enabling 14.9 g (yield: 90%) of Exemplified Compound (I-5) to be obtained.

### Example 3 (Synthesis of Exemplified Compounds (II-6), (I-4), and (I-52))

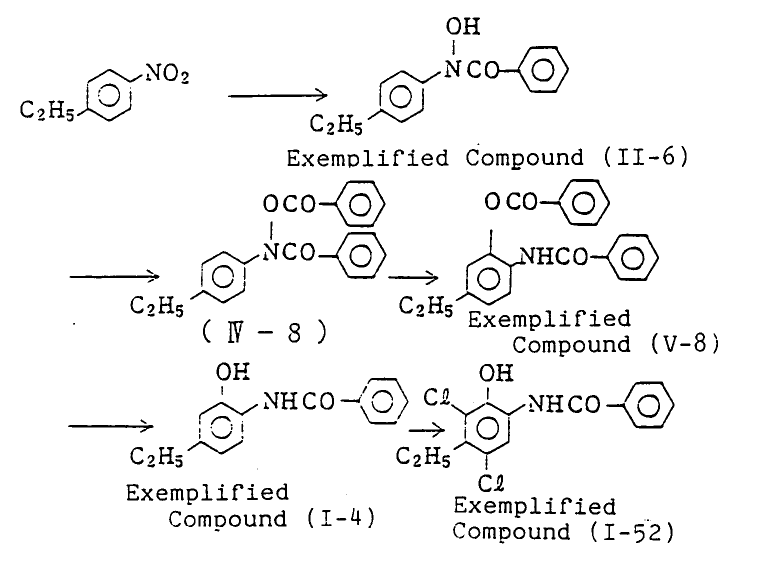

Similarly to Example 1, N-(4-ethylphenyl)hydroxylamine was synthesized, and then, in the place of acetyl chloride, 56 g (0.40 mol) of benzoyl chloride was used to carry out a similar reaction. After the organic layer and the aqueous layer were separated, the aqueous layer was extracted with ethyl acetate, twice; the extracted layer was combined with the organic layer, and the combined layer was washed with water and then with brine; then it was dried over magnesium sulfate, followed by filtration and condensation. The residue was purified by silica gel chromatography, thereby obtaining 63 g (65%) of Exemplified Compound (II-6) as crystals.

To a solution of 10 g (26.6 mmol) of Exemplified Compound (II-6) in 45 ml of toluene, was added 4.1 ml (29.3 mmol) of triethylamine, all at once, with cooling with water. Then a solution of 4.1 g (29.3 mmol) of benzoyl chloride in 5 ml of toluene was added thereto, dropwise, over about 10 min. After about 30 min of stirring of the reaction mixture, 30 ml of water was added thereto, and the toluene layer was separated. The toluene layer was washed with water once and then with brine once, and then it was dried over magnesium sulfate, followed by filtration and condensation, thereby enabling crystals of nearly pure Compound (IV-8) to be obtained quantitatively.

Melting point: 102 to 103 °C (recrystallized from acetonitrile)

### NMR (CDCl₃) of Compound (IV-8)

- δ: 1.20 (3H, t, J = 7.0), 2.60 (2H, q, J = 7.0) 7.13 (2H, d, J = 7.0), 7.22 to 7.40 (5H, m) 7.45 (2H, t, J = 7.0), 7.55 to 7.65 (3H, m) 8.10 (2H, d, J = 7.0)

The above Compound (IV-8) was heated as it was, without purifying it and without using any solvent, at 110 °C for 4 hours. As a result the raw material disappeared, and the product was Exemplified Compound (V-8). The reaction took place quantitatively.

### NMR (CDCl₃) of Exemplified Compound (V-8)

- δ: 1.28 (3H, t, J = 7.0), 2.68 (2H, q, J = 7.0) 7.12 (1H, d, J = 0.5), 7.18 (1H, dd, J = 7.0, 2.0) 7.38 (2H, t, J = 7.0), 7.42 to 7.55 (3H, m) 7.65 (1H, td, J = 7.0, 2.0), 7.75 (2H, dd, J = 7.0, 2.0) 7.95 (1H, brs), 8.17 (1H, d, J = 7.0) 8.22 (2H, dd, J = 7.0, 2.0)

The temperature was brought to room temperature (crystallization took place), and 20 ml of methanol was added, followed by heating, to dissolve the crystals. 27 ml (54 mmol) of an aqueous 2-N sodium hydroxide solution was added thereto, followed by stirring for about 3 hours. Upon acidifying it with 2-N hydrochloric acid, crystals were deposited, and water was added, to make the crystallization sufficient, followed by suction filtration and washing with water. The crystals were dried, to obtain 5.4 g (yield: 84%) of Exemplified Compound (I-4).

Melting point: 129 to 130 °C

### NMR (CDCl₃) of Exemplified Compound (I-4)

- δ: 1.22 (3H, t, J = 7.0), 2.60 (2H, q, J = 7.0) 6.74 (1H, dd, J = 7.0, 2.0), 6.92 (1H, d, J = 2.0) 7.05 (1H, d, J = 7.0), 7.49 to 7.55 (2H, m(triplet like)) 7.55 to 7.64 (1H, m(triplet like)), 7.90 (2H, dd, J = 7.0, 2.0), 8.08 (1H, brs), 8.72 (1H, brs)

For completion of the rearrangement reaction of Compound (IV-8), it took about 13 hours when the heating was carried out in toluene under reflux (at 110 °C), while it took about 30 min when the heating was carried out at 150 °C without using any solvent. When no solvent was used and benzoic acid was added in an amount of 1 equivalent, with the heating carried out at 110 °C, it took about 1 hour, while when no solvent was used, and benzoic acid was added in an amount of 0.1 equivalents, with the heating carried out at 110 °C, it took about 2 hours.

10 g (0.041 mol) of Exemplified Compound (I-4) was placed in 10 ml of acetic acid, followed by stirring. Although the Exemplified Compound (I-4) was not dissolved completely and was in a dispersed state, 7.0 ml (0.087 mol) of sulfuryl chloride was added thereto, all at once, at room temperature. The reaction liquid was at first solidified and as such it resisted stirring, but after a while it became stirable, and stirring was continued at that state for about 30 min; and then stirring was carried out at 50 °C for 30 min. After the temperature was brought to room temperature, water was added to the reaction mixture, and the deposited crystals were filtered and dried, thereby obtaining 12 g (yield: 94.5%) of nearly pure Exemplified Compound (I-52) as pale yellow crystals.

### NMR (CDCl₃) of Exemplified Compound (I-52)

- δ: 1.16 (3H, t, J = 7.0), 2.91 (2H, q, J = 7.0) 6.76 (1H, s), 7.48 to 7.62 (3H, m) 7.89 (2H, dd, J = 7.0, 2.0), 8.19 (1H, s) 8.27 (1H, brs)

### Example 4 (Synthesis of Exemplified Compounds (II-7) and (I-18))

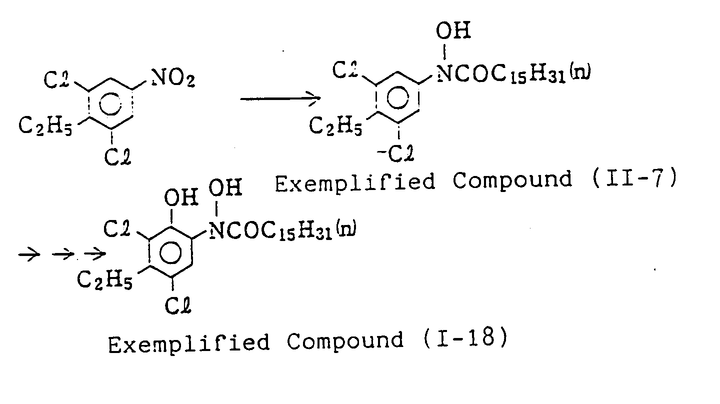

In a similar manner to that in Example 1, 22 g (0.1 mol) of 3,5-dichloro-4-ethylnitrobenzene (which was synthesized by chlorinating 4-ethylbenzene with chlorine and dechlorinating with copper, and was a compound described in JP-A No. 44552/1988) was reduced with zinc, and then it was reacted with 27.5 g (0.1 mol) of palmitoyl chloride in accordance with the Schotten-Baumann method, thereby enabling 33 g (75%) of Exemplified Compound (II-7) to be obtained.

The rearrangement reaction was carried out using 20 g (0.045 mol) of Exemplified Compound (II-7) by the method (5) in Example 2, i.e., the method using dichloroacetyl chloride, and hydrolysis was carried out at a stroke, thereby enabling 18 g (yield: 90%) of Exemplified Compound (I-18) to be obtained.

Melting point: 67 to 68 °C

### NMR (CDCl₃) of Exemplified Compound (I-18)

- δ: 0.89 (3H, t, J = 7.0), 1.15 (3H, t, J = 7.0) 1.20 to 1.40 (24H), 1.74 (2H, brq, J = 7.0) 2.42 (2H, t, J = 7.0), 2.90 (2H, q, J = 7.0) 7.16 (1H, s), 7.50 (1H, brs) 7.83 (1H, s)

### Example 5 (Synthesis of Exemplified Compounds (II-10), (I-8), and (I-15)

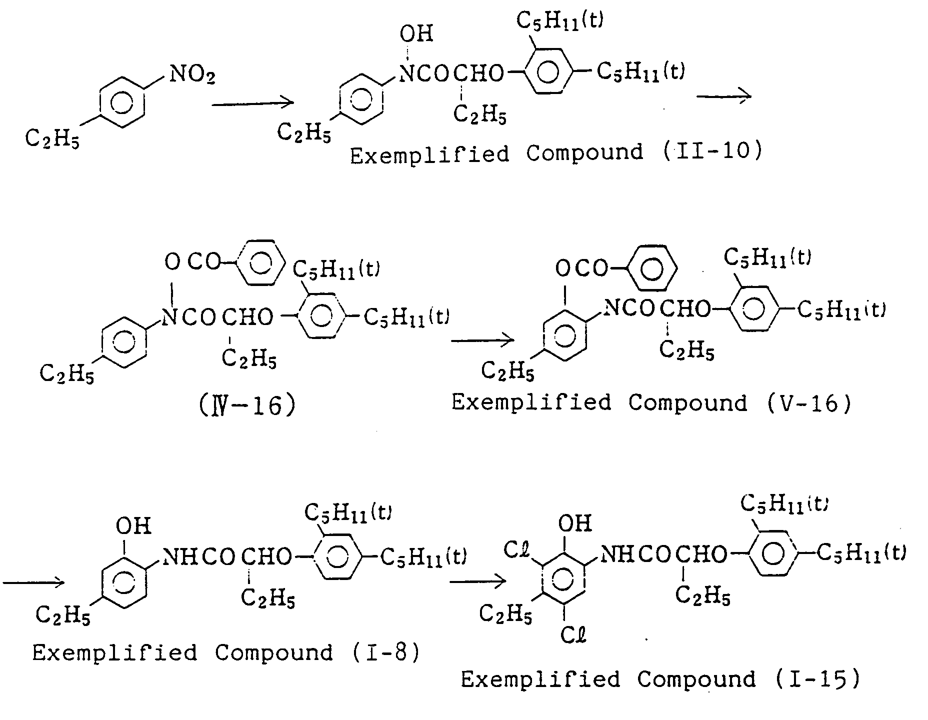

Similarly to Example 1, 40 g (0.26 mol) of 4-ethylnitrobenzene was reduced and was acylated by using 2-(2,4-di-t-amylphenoxy)butyloyl chloride, thereby enabling 93 g (80%) of Exemplified Compound (II-10) to be obtained.

In a similar manner to that of (2) of Example 2, using 44 g (0.1 mol) of Exemplified Compound (II-10), (IV-16) was synthesized, then the (IV-16) was mixed with 1 equivalent of benzoic acid without purifying the (IV-16); the resulting mixture was heated at 120 °C for about 4 hours, to obtain Exemplified Compound (V-16), and Exemplified Compound (V-16) was deacylated with hydroxylamine, thereby enabling 41 g (93%) of Exemplified Compound (I-8) to be obtained.

41 g (0.093 mol) of the thus obtained Exemplified Compound (1-8) was dissolved in dichloromethane, and 12.4 g (0.093 mol) of N-chlorosuccinic acid imide was added to the solution, at room temperature. Then 18.2 ml (0.093 mol) of a 28% solution of sodium methoxide in methanol was added thereto, and again 12.4 g (0.093 mol) of N-chlorosuccinic acid imide was added, at room temperature. After about one hour of stirring of the reaction mixture, an extraction operation was carried out and crystallization from acetonitrile was performed, thereby enabling 45 g (95%) of Exemplified Compound (I-15) to be obtained.

Melting point: 144 to 145 °C

### Example 6 (Synthesis of Exemplified Compounds (II-11) and (I-15)

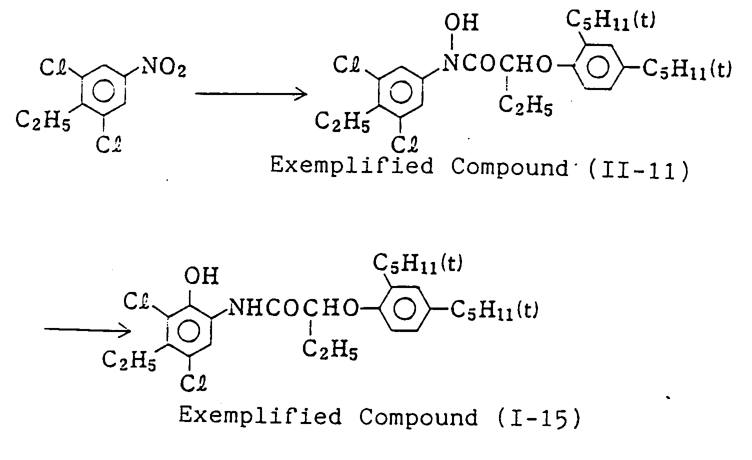

Similarly to Example 4, 22 g (0.1 mol) of 3,5-dichloro-4-ethylnitrobenzene was reduced with zinc, to obtain a hydroxylamine compound, and then it was acylated with 2-(2,4-di-t-amylphenoxy)butyloyl chloride, thereby enabling 41 g (yield: 81%) of Exemplified Compound (II-11) to be obtained.

All of the thus obtained Exemplified Compound (II-1) was dissolved in acetonitrile; rearrangement reaction was carried out using dichloroacetyl chloride, in a similar manner to that shown in (5) of Example 2, and hydrolysis was effected at a stroke, thereby enabling 38 g (93%) of Exemplified Compound (I-15) to be obtained.
Melting point: 144 to 145 °C

### Example 7 (Synthesis of Exemplified Compounds (II-17) and (I-27)

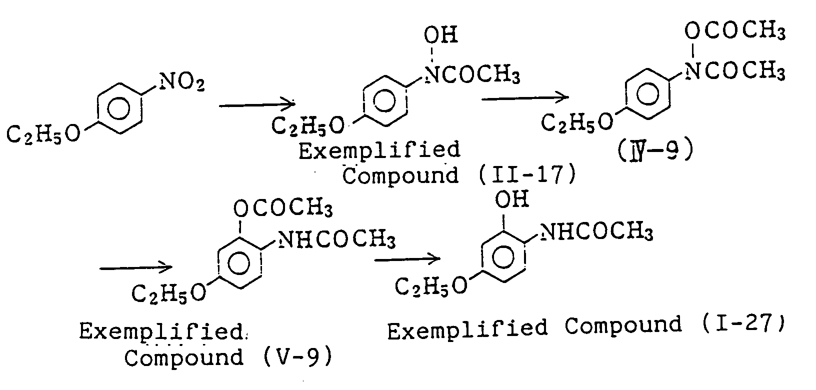

Similarly to Example 1, after 30 g (0.179 mol) of 4-ethoxynitrobenzene was reduced with zinc, the obtained product was acetylated with acetyl chloride in accordance with the Schotten-Baumann method, thereby yielding 24.5 g (70%) of Exemplified Compound (II-17).

20 g (0.102 mol) of the thus obtained Exemplified Compound (II-17) was acetylated with acetyl chloride/triethylamine, to obtain Compound (IV-9); the Compound (IV-9) was heated at 120 °C for 5 hours without using any solvent, to cause rearrangement reaction to take place, to obtain Exemplified Compound (V-9), and the Exemplified Compound (V-9) was hydrolyzed with an aqueous sodium hydroxide solution, thereby enabling 19 g (95%) of Exemplified Compound (I-27) to be obtained.

### Example 8 (Synthesis of Exemplified Compounds (II-19) and (I-28)

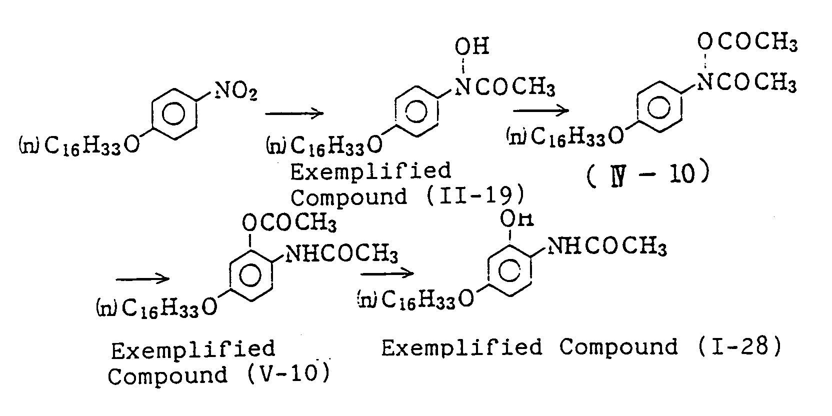

40 g (0.11 mol) of 4-hexadecyloxynitrobenzene could not be reduced with zinc, because the compound would not disslove at all in a water-methanol system. Therefore, 40 g (0.11 mol) of 4-hexadecyloxynitrobenzene was reduced to a hydroxylamine compound, in accordance with the method described in "Synthesis," 1984, page 938; that is, using conditions of NH₂NH₂^{·}H₂O/Raney Ni/C₂H₅OH/ClCH₂CH₂Cl. After the reduction, the hydroxylamine was acetylated with acetyl chloride in accordance with the Schotten-Baumann method, thereby yielding 34.5 g (80%) of Exemplified Compound (II-19).

Similarly to Example 9, using 30 g (0.077 mol) of Exemplified Compound (II-19), the acetylation-rearrangement reaction was carried out, to obtain Exemplified Compound (V-10), and the Exemplified Compound (V-10) was hydrolyzed, thereby enabling 27.3 g (91%) of Exemplified Compound (I-28) to be obtained.

### Example 9 (Synthesis of Exemplified Compounds (II-20) and (I-29)

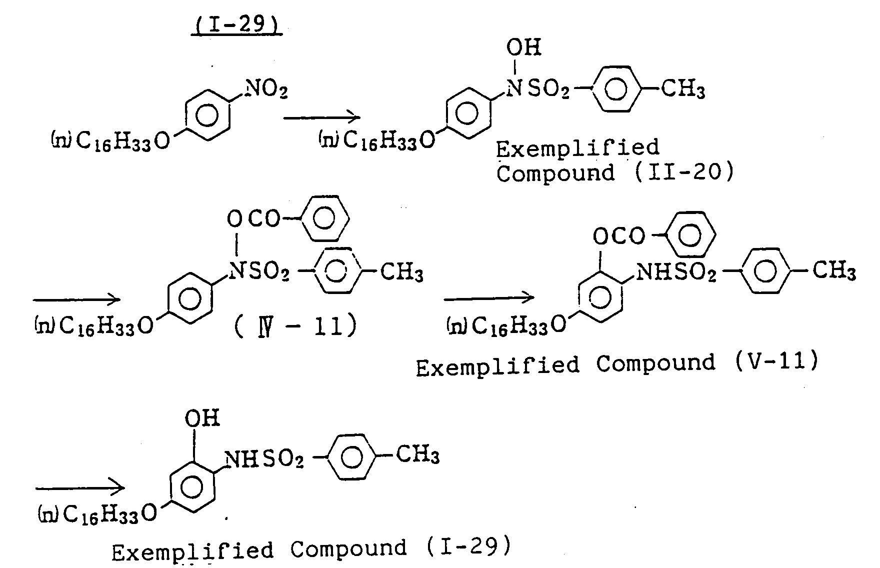

Similarly to Example 8, 40 g (0.11 mol) of 4-hexadecyloxynitrobenzene was reduced to a hydroxylamine; water was added to the reaction mixture, to separate a dichloroethane layer, and the reaction product was reacted with 22.9 g (0.12 mol) of p-toluenesulfonyl chloride using 8.9 ml (0.11 mol) of pyridine as a base, thereby yielding 41.6 g (75%) of Exemplified Compound (II-20).

20 g (0.040 mol) of the Exemplified Compound (II-20) was dissolved in 100 ml of toluene, and 6.3 ml (0.045 mol) of triethylamine was added to the solution. Then, a solution of 7.0 g (0.05 mol) of benzoyl chloride in 5 ml of toluene was added, dropwise. After 30 min of stirring of the resulting mixture, water was added, to separate a toluene layer, and the toluene layer was washed with water and then with an aqueous saturated table salt solution. The thus obtained toluene solution containing Compound (IV-11) was heated as it was under reflux, to undergo a reaction for about 5 hours, to obtain Exemplified Compound (V-11). Thereafter, 80 ml or little of the toluene was distilled off under reduced pressure; then 50 ml of methanol and 50 ml (0.1 mol) of an aqueous 2-N sodium hydroxide solution were added thereto, and the reaction mixture was stirred for about 1 hour at room temperature. When the reaction mixture was rendered acidic with hydrochloric acid, crystals were deposited, and the crystals were then filtered and dried, thereby obtaining 17 g (85%) of Exemplified Compound (I-29).

### NMR (CDCl₃) of Exemplified Compound (I-29)

- δ: 0.89 (3H, t, J = 7.0), 1.18 to 1.50 (26H) 1.74 (2H, quintet, J = 7.0), 2.41 (3H, s) 3.86 (2H, t, J = 7.0), 6.00 (1H, s) 6.21 (1H, dd, J = 7.0, 2.0), 6.38 (1H, d, J = 7.0) 6.50 (1H, dd, J = 2.0), 7.24 (2H, d, J = 8.0) 7.26 (1H, s), 7.59 (2H, d, J = 8.0)

### Example 10 (Synthesis of Exemplified Compounds (II-26) and (I-31)

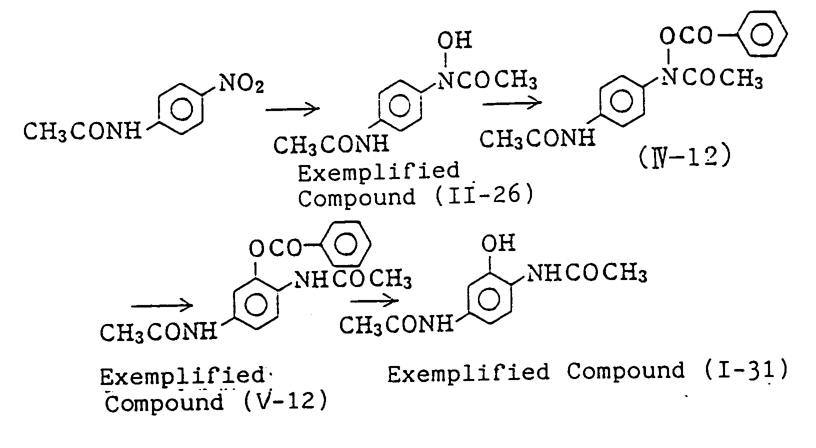

Similarly to Example 1, 18 g (0.10 mol) of 4-acetylaminonitrobenzene was reduced and acetylated, thereby enabling 15 g (72%) of Exemplified Compound (II-26) to be obtained.

10 g (0.048 mol) of the Exemplified Compound (II-26) was treated in a similar manner to that in (2) of Example 2, to obtain Compound (IV-12); benzoic acid was added to the Compound (IV-12), and the mixture was heated at 110 °C for 8 hours, to obtain Exemplified Compound (V-12). The Exemplified Compound (V-12) was hydrolyzed with hydroxylamine, thereby yielding 8.5 g (85%) of Exemplified Compound (I-31).

### NMR (CDCl₃/DMSO-d₆) of Exemplified Compound (I-31)

- δ: 2.10 (3H, s), 2.19 (3H, s)
6.93 (1H, dd, J = 7.0, 2.0)
7.34 (1H, d, J = 2.0)
7.43 (1H, d, J = 7.0)
7.64 (1H, brs)
9.09 (1H, brs)
9.45 (1H, brs)

### Example 11 (Synthesis of Exemplified Compound (I-18) from Exemplified Compound (I-5)

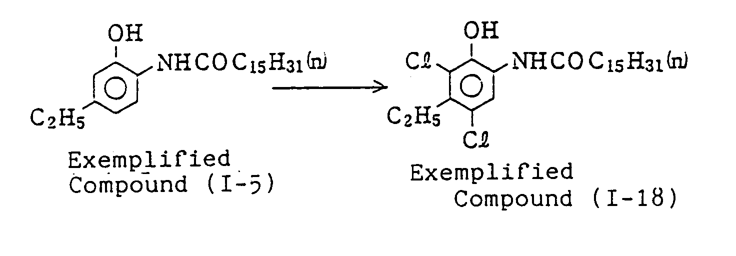

A solution of 50 g (0.133 mol) of Exemplified Compound (I-5) in 250 ml of acetic acid was stirred at room temperature. At this stage, the Exemplified Compound (I-5) was not completely dissolved and was in a dispersed state. 24 ml (0.293 mol) of sulfuryl chloride was added, dropwise, thereto over about 20 min. The reaction liquid turned gradually transparent and crystals were gradually deposited, during which a little heat was generated. After about 1 hour of stirring thereof, 250 ml of water was added, and the deposited crystals were filtered, washed with water, and dried, thereby enabling 59 g (99.8%) of Exemplified Compound (I-18) to be obtained.
Melting point: 66.5 to 68 °C (recrystallized from acetonitrile/dichloromethane)

The NMR showed exactly the same spectrum as that obtained in Example 4.

Now it is shown that the compound represented by formula (VII) is useful as a cyan coupler for color photography.

First, Exemplified Compound (I-13) corresponding to formula (VII) was chlorinated by the method shown in Example (11), to be converted to Exemplified Compound (I-19).

Then, the preparation of a multilayer color printing paper (Sample 1), described in Example 1 shown in JP-A No. 289518/1994, was repeated, but the cyan coupler (ExC) in the fifth layer, in an amount of 0.33 g/m², was changed to Exemplified Compound (I-18), in an amount of 0.31 g/m², thereby preparing a multilayer color printing paper (101).

Samples (102), (103), (104), and (105) were prepared by replacing the cyan coupler (Exemplified Compound (I-18)) in the multilayer color printing paper (101) with an equimolar amount of Exemplified Compound (I-19), (I-20), or (I-50).

These Samples were subjected to three-color separation exposure through an optical wedge and were processed through the steps described in Example 1 of the above JP-A No. 289518/1994.

The dye image densities of the three colors of the processed Samples were measured by a Macbeth densitometer status AA filter, and it was found that the extent of the color mixing of cyan into the magenta dye image varied depending on the Samples. The results are shown in the following Table.

**Table 1**

| Deffusion of cyan coupler into magenta layer | | |
|---|---|---|
| Sample | Coupler | Cyan dye density (D_{C}) in the magenta dye image (D_{M} = 1.0)* |
| 101 | I-18 | 0.03 |
| 102 | I-19 | 0.02 |
| 103 | I-20 | 0.14 |
| 104 | I-50 | 0.10 |

| | | |
|---|---|---|
| * The magenta dye density (D_{M}) and cyan dye density (D_{C}) in the magenta dye image is the value obtained by subtracting the magenta dye density and the cyan dye density possessed by the base (white). | | |

As is shown in Table 1, in Samples (101) and (102), wherein Exemplified Compound (I-18) or (I-19), that is, the couplers according to the present invention, are used, the cyan color mixing into the magenta dye image is less. It is shown that, in Comparative Samples (103) and (104), whose couplers have an aliphatic hydrocarbon carbonylamino group with less than 15 carbon atoms, the cyan coupler diffuses into another layer (magenta); whereas the coupler according to the present invention, in which the carbon number is 15 or more, has satisfactory diffusion resistance. Thus it can be seen that the compound of formula (VII) is useful as a precursor of a cyan coupler for color photography.

Further, a dye-releasing agent was synthesized using, as a starting raw material, Exemplified Compound (I-28) obtained in the above Example, and the photographic performance thereof was evaluated based on the Example described in JP-A No. 16131/1981. The results showed the similar photographic performance as that of the dye-releasing agent synthesized by the conventional synthetic method.

## Claims

1. A method for producing 5-substituted-2-acylaminophenols represented by the following formula (I), comprising partially deacylating a compound represented by the following formula (V): 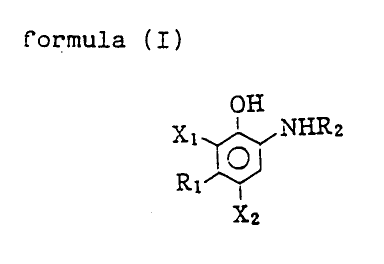 in formula (I), R₁ represents an aliphatic hydrocarbon group, an aliphatic hydrocarbon oxy group, an aryloxy group, an acylamino group, a disubstituted amino group, or a hydroxyl group; R₂ represents an acyl group; and X₁ and X₂ each represent a hydrogen atom or a halogen atom, 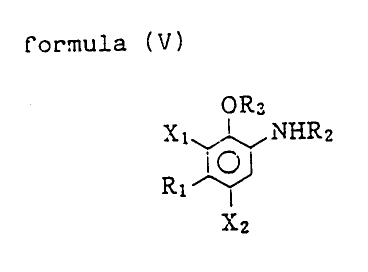 wherein R₁, R₂, X₁, and X₂ each have the same meaning as defined in formula (I), and R₃ represents an acyl group.

2. The method as claimed in claim 1, wherein the deacylation is carried out in the presence of hydroxylamine.

3. The method as claimed in claim 1 or 2, wherein the compound represented by formula (V) is a compound obtained by subjecting a compound represented by the following formula (IV) to acyloxyl rearrangement: 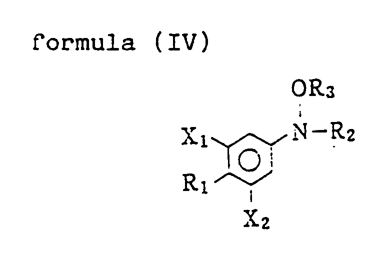 in formula (IV), R₁, R₂, R₃, X₁, and X₂ each have the same meaning as defined in formula (V).

4. The method as claimed in claim 1, 2, or 3, wherein the said acyloxyl rearrangement is carried out in the presence of acid catalyst(s).

5. The method as claimed in claim 3, or 4, wherein the compound represented by formula (IV) is a compound obtained by acylating N-acyl-N-substituted phenylhydroxylamines represented by formula (II): 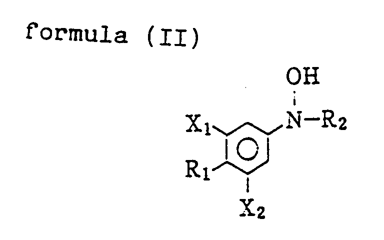 in formula (II), R₁, R₂, X₁, and X₂ each have the same meaning as defined in formula (IV).

6. A method for producing a compound represented by the following formula (XI), comprising acylating a compound represented by the following formula (IX), to form a compound represented by the following formula (X), and subjecting the compound of formula (X) to an acyloxyl rearrangement reaction to give a compound of formula (XI): 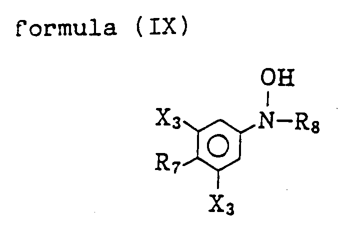 wherein R₇ represents an aliphatic hydrocarbon group or an aliphatic hydrocarbon oxy group having 2 to 18 carbon atoms, or an acylamino group; R₈ represents an acyl group; and X₃ represents a hydrogen atom or a halogen atom, 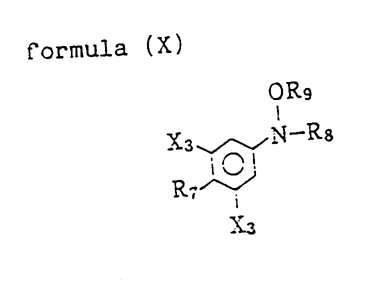 wherein R₇, R₈, and X₃ each have the same meaning as defined in formula (IX), and R₉ represents an acyl group, 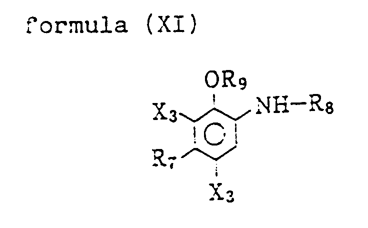 wherein R₇, R₈, R₉, and X₃ each have the same meaning as defined in formulae (IX) and (X).

7. An N-acyl-N-(4-alkylphenyl)hydroxylamine compound represented by the following formula (VIII): 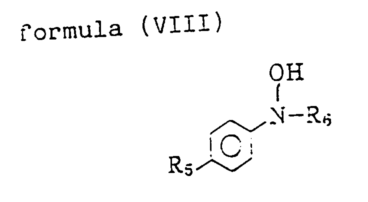 wherein R₅ represents an alkyl group having 2 to 9 carbon atoms, and R₆ represents an aliphatic carbonyl group having 15 to 22 carbon atoms.

8. Use of an N-acyl-N-(4-alkylphenyl)hydroxylamine compound represented by formula (VIII) in producing a 5-alkyl-2-acylaminophenol compound represented by formula (VII): 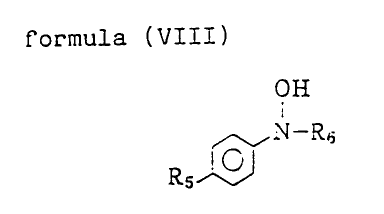 wherein R₅ represents an alkyl group having 2 to 9 carbon atoms, and R₆ represents an aliphatic carbonyl group having 15 to 22 carbon atoms, 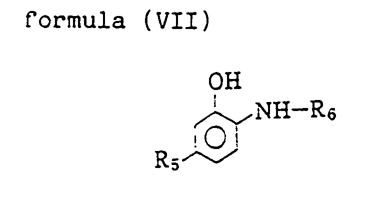 wherein R₅ represents an alkyl group having 2 to 9 carbon atoms, and R₆ represents an aliphatic carbonyl group having 15 to 22 carbon atoms.

## Patentansprüche

1. Verfahren zur Herstellung von 5-substituierten 2-Acylaminophenolen, dargestellt durch die folgende Formel (I), umfassend die partielle Deacylierung einer durch die folgende Formel (V) dargestellten Verbindung: 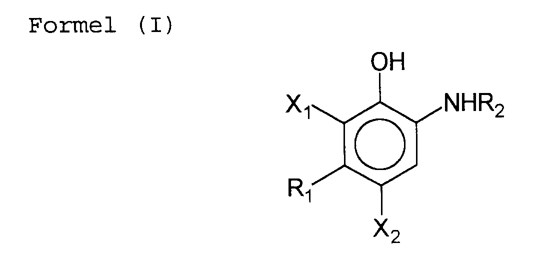 worin in Formel (I) R₁ eine aliphatische Kohlenwasserstoffgruppe, eine aliphatische Kohlenwasserstoffoxygruppe, eine Aryloxygruppe, eine Acylaminogruppe, eine disubstituierte Aminogruppe oder eine Hydroxylgruppe darstellt; R₂ eine Acylgruppe darstellt; und X₁ und X₂ jeweils ein Wasserstoffatom oder ein Halogenatom darstellen, 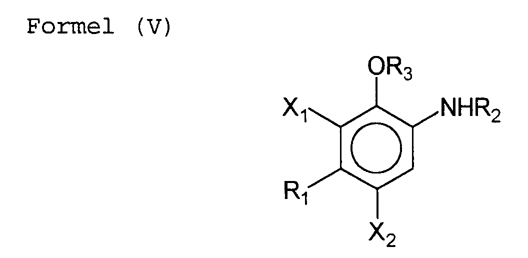 worin R₁, R₂, X₁ und X₂ jeweils die gleiche Bedeutung wie in Formel (I) definiert haben und R₃ eine Acylgruppe darstellt.

2. Verfahren gemäss Anspruch 1, worin die Deacylierung in Gegenwart von Hydroxylamin durchgeführt wird.

3. Verfahren gemäss Anspruch 1 oder 2, worin die durch die Formel (V) dargestellte Verbindung eine Verbindung ist, die erhalten wird durch Unterwerfen einer durch die folgende Formel (IV) dargestellten Verbindung einer Acyloxy-Umlagerung: 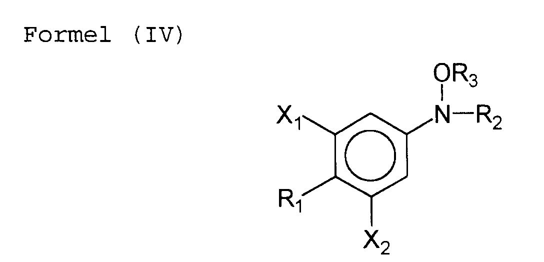 worin in Formel (IV) R₁, R₂, R₃, X₁ und X₂ jeweils die gleiche Bedeutung wie in Formel (V) definiert haben.

4. Verfahren gemäss Anspruch 1, 2 oder 3, worin die Acyloxy-Umlagerung in Gegenwart von Säurekatalysator(en) durchgeführt wird.

5. Verfahren gemäss Anspruch 3 oder 4, worin die durch die Formel (IV) dargestellte Verbindung eine Verbindung ist, die erhalten wird durch Acylieren von durch die Formel (II) dargestellten N-Acyl-N-substituierten Phenylhydroxylaminen: 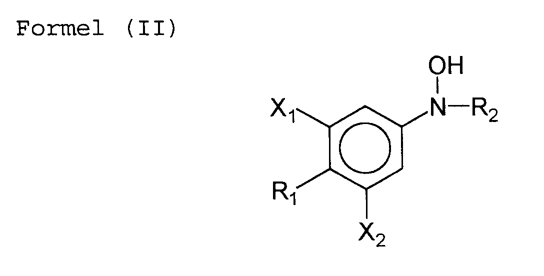 worin in Formel (II) R₁, R₂, X₁ und X₂ jeweils die gleiche Bedeutung wie in Formel (IV) definiert haben.

6. Verfahren zur Herstellung einer durch die folgende Formel (XI) dargestellten Verbindung, umfassend die Acylierung einer durch die folgende Formel (IX) dargestellten Verbindung, um eine durch die folgende Formel (X) dargestellte Verbindung zu bilden, und Unterwerfen der Verbindung der Formel (X) einer Acyloxy-Umlagerungsreaktion, um eine Verbindung der Formel (XI) zu ergeben: 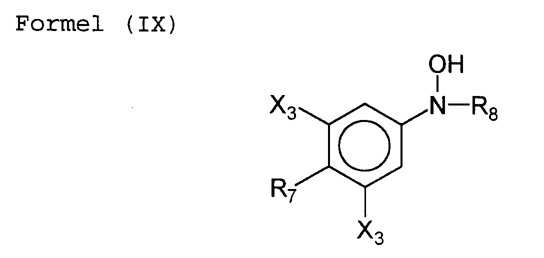 worin R₇ eine aliphatische Kohlenwasserstoffgruppe oder eine aliphatische Kohlenwasserstoffoxygruppe mit 2 bis 18 Kohlenstoffatomen oder eine Acylaminogruppe darstellt; R₈ eine Acylgruppe darstellt; und X₃ ein Wasserstoffatom oder ein Halogenatom darstellt; 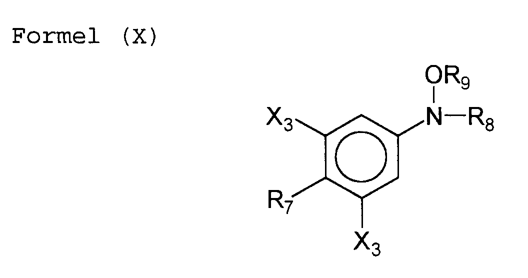 worin R₇, R₈ und X₃ jeweils die gleiche Bedeutung wie in Formel (IX) definiert haben und R₉ eine Acylgruppe darstellt; 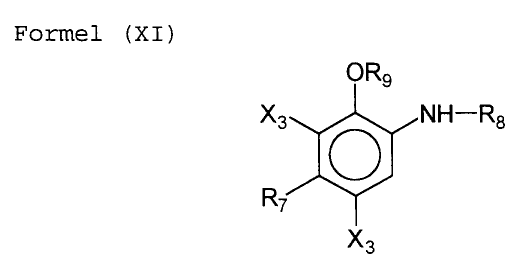 worin R₇, R₈, R₉ und X₃ jeweils die gleiche Bedeutung wie in den Formeln (IX) und (X) definiert haben.

7. N-Acyl-N-(4-alkylphenyl)hydroxylamin-Verbindung, dargestellt durch die folgende Formel (VIII): 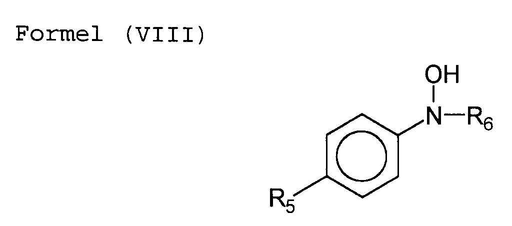 worin R₅ eine Alkylgruppe mit 2 bis 9 Kohlenstoffatomen darstellt und R₆ eine aliphatische Carbonylgruppe mit 15 bis 22 Kohlenstoffatomen darstellt.

8. Verwendung einer durch die Formel (VIII) dargestellten N-Acyl-N-(4-alkylphenyl)hydroxylamin-Verbindung bei der Herstellung einer durch die Formel (VII) dargestellten 5-Alkyl-2-acylaminophenol-Verbindung: 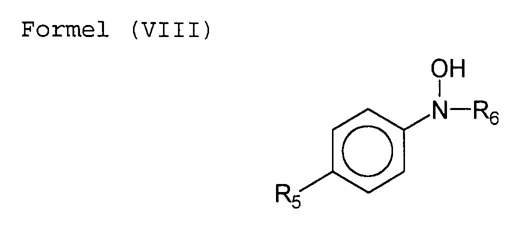 worin R₅ eine Alkylgruppe mit 2 bis 9 Kohlenstoffatomen darstellt und R₆ eine aliphatische Carbonylgruppe mit 15 bis 22 Kohlenstoffatomen darstellt, 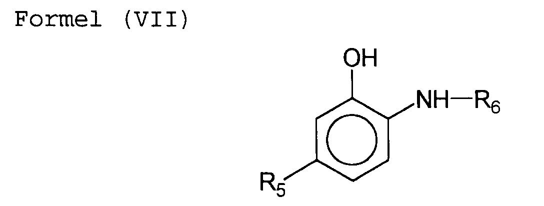 worin R₅ eine Alkylgruppe mit 2 bis 9 Kohlenstoffatomen darstellt und R₆ eine aliphatische Carbonylgruppe mit 15 bis 22 Kohlenstoffatomen darstellt.

## Revendications

1. Procédé pour préparer des 2-acylaminophénols substitués en position 5, représentés par la formule suivante (I), comprenant de désacyler partiellement un composé représenté par la formule suivante (V) : 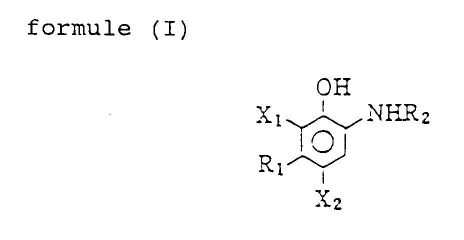 dans la formule (I), R₁ représente un groupe hydrocarbure aliphatique, un groupe hydrocarbure-oxy aliphatique, un groupe aryloxy, un groupe acylamino, un groupe amino di-substitué ou un groupe hydroxyle; R₂ représente un groupe acyle; et X₁ et X₂ représentent chacun un atome d'hydrogène ou un atome d'halogène, 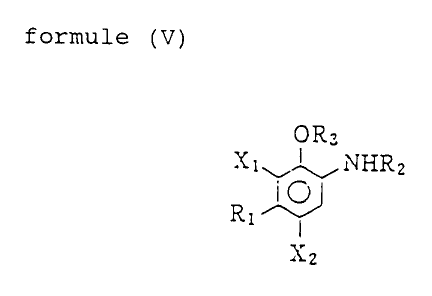 dans laquelle R₁, R₂, X₁ et X₂ ont chacun la même signification que celle définie pour la formule (I) et R₃ représente un groupe acyle.

2. Procédé selon la revendication 1, dans lequel la désacylation se fait en présence d'hydroxylamine.

3. Procédé selon la revendication 1 ou 2, dans lequel le composé représenté par la formule (V) est un composé obtenu en soumettant un composé représenté par la formule (IV) suivante à un réarrangement du groupe acyloxy : 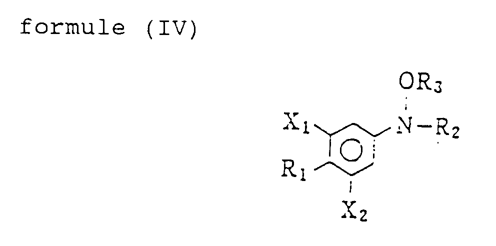 dans la formule (IV), R₁, R₂, R₃, X₁ et X₂ ont chacun la même signification que celle définie pour la formule (V).

4. Procédé selon la revendication 1, 2 ou 3, dans lequel ledit réarrangement du groupe acyloxy se fait en présence d'un ou de plusieurs catalyseurs acides.

5. Procédé selon la revendication 3 ou 4, dans lequel le composé représenté par la formule (IV) est un composé obtenu par l'acylation de N-acyl-N-substitué-phénylhydroxylamines représentées par la formule (II) : 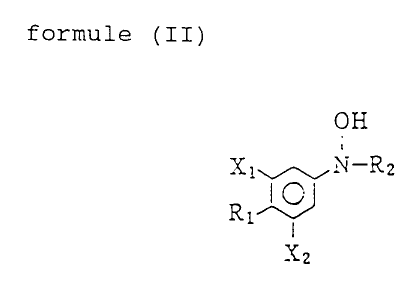 dans la formule (II), R₁, R₂, X₁ et X₂ ont chacun la même signification que celle définie pour la formule (IV).

6. Procédé pour préparer un composé représenté par la formule suivante (XI), comprenant d'acyler un composé représenté par la formule (IX) pour former un composé représenté par la formule suivante (X) et de soumettre le composé de la formule (X) à une réaction de réarrangement du groupe acyloxy pour donner un composé de la formule (XI) : 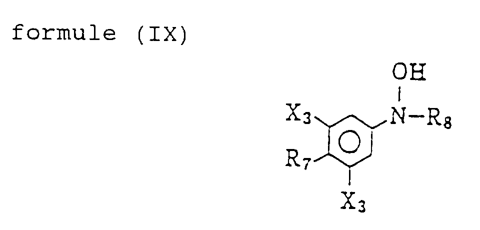 dans laquelle R₇ représente un groupe hydrocarbure aliphatique ou un groupe hydrocarbure-oxy aliphatique ayant 2 à 18 atomes de carbone, ou un groupe acylamino; R₈ représente un groupe acyle; et X₃ représente un atome d'hydrogène ou un atome d'halogène, 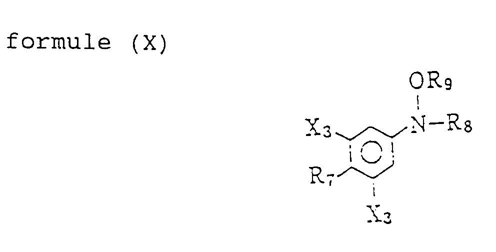 dans laquelle R₇, R₈ et X₃ ont chacun la même signification que celle définie pour la formule (IX) et R₉ représente un groupe acyle, 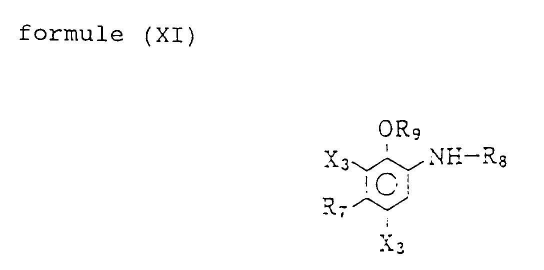 dans laquelle R₇, R₈, R₉ et X₃ ont chacun la même signification que celle définie pour les formules (IX) et (X).

7. Composé N-acyl-N-(4-alkylphényl)hydroxy(amine représenté par la formule (VIII) suivante : 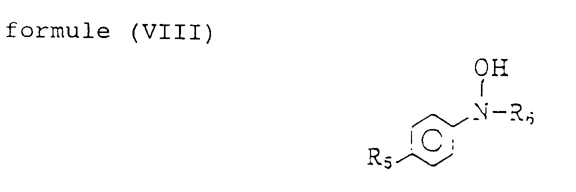 dans laquelle R₅ représente un groupe alkyle ayant 2 à 9 atomes de carbone et R₆ représente un groupe carbonyle aliphatique ayant 15 à 22 atomes de carbone.

8. Utilisation d'un composé N-acyl-N-(4-alkylphényl)-hydroxylamine représenté par la formule (VIII) pour préparer un composé 5-alkyl-2-acylaminophénol représenté par la formule (VII) : 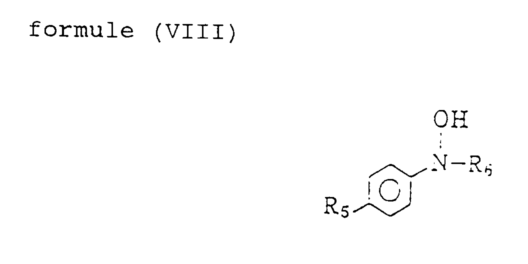 dans laquelle R₅ représente un groupe alkyle ayant 2 à 9 atomes de carbone et R₆ représente un groupe carbonyle aliphatique ayant de 15 a 22 atomes de carbone, 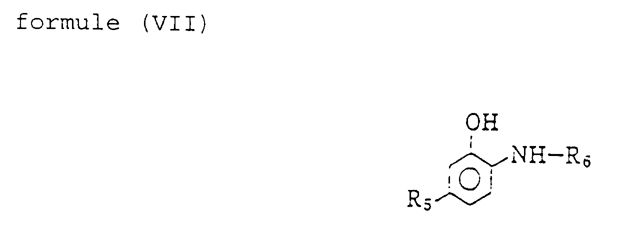 dans laquelle R₅ représente un groupe alkyle ayant de 2 à 9 atomes de carbone et R₆ représente un groupe carbonyle aliphatique ayant de 15 à 22 atomes de carbone.
